# EUROPÄISCHE PATENTSCHRIFT

(11) **EP 2 956 533 B1**
(45) Veröffentlichungstag und Bekanntmachung des Hinweises auf die Patenterteilung: **17.04.2019**
(21) Anmeldenummer: 14703861.6
(22) Anmeldetag: 12.02.2014
(51) Int. Cl.: C11D 3/04, C12N 9/96, C11D 3/386

(54) **FLÜSSIGES WASCH- ODER REINIGUNGSMITTEL MIT VERBESSERTER ENZYMSTABILITÄT**
LIQUID WASHING OR CLEANING PRODUCT HAVING IMPROVED ENZYME STABILITY
LIQUIDE DE LAVAGE OU DE NETTOYAGE AVEC UNE STABILITÉ AMÉLIORÉE DES ENZYMES

(30) Priorität: 14.02.2013 DE 102013202450
(43) Veröffentlichungstag der Anmeldung: 23.12.2015
(73) Patentinhaber: Henkel AG & Co. KGaA, 40589 Düsseldorf (DE)
(72) Erfinder: PEGELOW, Ulrich, 40597 Düsseldorf (DE); BUISKER, Detlef, 45134 Essen (DE); RASCHKE, Ines, 40764 Langenfeld (DE)
(86) Internationale Anmeldenummer: PCT/EP2014/052677
(87) Internationale Veröffentlichungsnummer: WO 2014/124948

(56) Entgegenhaltungen:
- EP-A1- 0 072 581
- EP-A1- 2 679 679
- WO-A1-02/02727
- WO-A1-96/21716
- WO-A1-96/41859
- WO-A1-2011/110593
- WO-A1-2011/147665
- WO-A2-02/08398
- DE-A1- 10 062 858
- US-A- 5 510 052
- US-A1- 2009 165 821

## Beschreibung

Die vorliegende Erfindung betrifft enzymhaltige flüssige Wasch- oder Reinigungsmittel, insbesondere Handgeschirrspülmittel, mit verbesserter Enzymstabilität.

Übliche Wasch- oder Reinigungsmittel enthalten Tenside zur Entfernung von Schmutz und Flecken. In der Regel werden hierbei Kombinationen aus mehreren Tensiden, insbesondere aus der Gruppe der anionischen, nichtionischen, kationischen und amphoteren Tenside verwendet. Diese Tenside allein sind häufig nicht in der Lage, Schmutz und Flecken hinreichend zu entfernen, so dass in modernen Wasch- oder Reinigungsmitteln weitere Hilfsstoffe eingesetzt werden. Zu diesen weiteren Hilfsstoffen gehören Enzyme verschiedener Arten wie Proteasen, Amylasen, Cellulasen, Mannanasen oder Pektatlyasen. Dem Fachmann sind weitere Enzymklassen bekannt. Insbesondere hydrolytische Enzyme wie Proteasen, Amylasen oder Lipasen sind wegen ihrer unmittelbar reinigenden Wirkung Bestandteil zahlreicher Textil- oder Geschirrreinigungsmittel.

Die für den Endverbraucher entscheidende Reinigungswirkung der in Wasch- oder Reinigungsmitteln eingesetzten Enzyme wird neben der Enzymstruktur in wesentlichem Maße auch durch die Art der Konfektionierung dieser Enzyme und ihrer Stabilisierung gegen Umwelteinflüsse bestimmt.

Wasch- oder reinigungsaktive Enzyme werden sowohl in fester als auch in flüssiger Form konfektioniert. Zur Gruppe der festen Enzymzubereitungen zählen insbesondere die aus mehreren Inhaltsstoffen bestehenden Enzymgranulate, die ihrerseits vorzugsweise in feste Wasch- oder Reinigungsmittel eingearbeitet werden. Flüssige oder gelförmige Wasch- oder Reinigungsmittel enthalten im Gegensatz hierzu häufig flüssige Enzymzubereitungen, wobei diese, anders als die Enzymgranulate gegen äußere Einflüsse weit weniger geschützt sind.

Zur Erhöhung der Stabilität derartiger Enzym-haltiger flüssiger Wasch- oder Reinigungsmittel wurden eine Reihe unterschiedlicher Schutzmaßnahmen vorgeschlagen. So lehrt beispielsweise die deutsche Patentanmeldung DE 20 38 103 (Henkel) die Stabilisierung von Enzym-haltigen Geschirrspülmitteln durch Saccharide, während in dem europäischen Patent EP 646 170 B1 (Procter & Gamble) Propylenglykol zur Enzymstabilisierung in flüssigen Reinigungsmitteln offenbart wird.

Als reversible Proteaseinhibitoren sind im Stand der Technik Polyole, insbesondere Glycerin und 1,2-Propylenglycol beschrieben. Eine entsprechende technische Offenbarung findet sich beispielsweise in der internationalen Anmeldung WO 02/08398 A2 (Genencor).

In der deutschen Patentanmeldung DE 100 62 858 A1 wird die Verwendung von Alkalisalzen niederer Carbonsäuren als Enzymstabilisatoren erwähnt.

Die US Patentschrift 5,510,052 nennt ebenfalls Alkalisalze niederer Carbonsäuren als Enzymstabilisatoren. Kaliumacetat wird in keiner dieser Druckschriften erwähnt.

Ferner sind Borax, Borsäuren, Boronsäuren oder deren Salze oder Ester als Stabilisatoren bekannt. Darunter sind vor allem Derivate mit aromatischen Gruppen, etwa ortho-, meta- oder para-substituierte Phenylboronsäuren zu erwähnen, insbesondere 4-Formylphenyl-Boron-säure (4-FPBA) beziehungsweise die Salze oder Ester der genannten Verbindungen. Letztgenannte Verbindungen als Enzymstabilisatoren sind beispielsweise offenbart in der internationalen Patentanmeldung WO 96/41859 A1 (Novo Nordisk). Allerdings weisen beispielsweise Borsäuren und Borsäurederivate oftmals den Nachteil auf, dass sie mit anderen Inhaltsstoffen einer Zusammensetzung, insbesondere Wasch- bzw. Reinigungsmittelinhaltsstoffen, unerwünschte Nebenprodukte bilden, so dass diese in den betreffenden Mitteln nicht mehr für den erwünschten Reinigungszweck zur Verfügung stehen oder sogar als Verunreinigung auf dem Waschgut zurückbleiben. Ferner werden Borsäuren bzw. Borate unter Umweltaspekten als nachteilig betrachtet.

Eine weitere Möglichkeit der Stabilisierung von Enzymen in wässrigen Reinigungsmitteln besteht in der Verwendung von Calciumsalzen wie Calciumformiat, Calciumacetat oder Calciumpropionat (US Patent 4,318, 818; Procter & Gamble). Aus der internationalen Patentanmeldung WO 2011/147665 sind flüssige Reinigungsmittel bekannt, welche 20 bis 70 Gew.-% Wasser, mindestens eine Amylase-Zubereitung, mindestens eine Calciumionenquelle und Milchsäure oder ein Milchsäuresalz enthalten. Salze mehrwertiger Kationen wie Calciumkationen führen jedoch in wässrigen Systemen, insbesondere in manuellen Geschirrspülmitteln häufig zu Trübungen während der Lagerung. Dieser negative Effekt verstärkt sich bei der Lagerung bei tiefen Temperaturen. Dadurch sind die möglichen Einsatzkonzentrationen limitiert, so dass keine ausreichende enzymstabilisierende Wirkung garantiert werden kann.

Die Aufgabe der vorliegenden Erfindung bestand somit daher darin, eine Methode zur Stabilisierung von Enzymen in flüssigen Wasch- oder Reinigungsmitteln bereitzustellen, die zumindest einen Nachteil aus dem Stand der Technik wenigstens verbessert. Die Aufgabe bestand außerdem darin, flüssige Wasch- oder Reinigungsmittel mit einer verbesserten Enzymstabilität bereitzustellen.

Überraschend wurde gefunden, dass die Zugabe von Kaliumsalzen die Stabilität von Enzymen in flüssigen Wasch- oder Reinigungsmitteln, insbesondere in Handgeschirrspülmitteln, gegenüber herkömmlichen Wasch- und Reinigungsmitteln deutlich verbessert. So sind erfindungsgemäß Kaliumsalze in solchen Einsatzkonzentrationen einsetzbar, die eine ausreichend gute Stabilisierung der Enzyme erlauben, aber gleichwohl keine wesentliche Trübung der Zusammensetzung bewirken. So wurde in den erfindungsgemäßen Wasch- und Reinigungsmitteln mit Kaliumsalzen beispielsweise selbst nach einer vierwöchigen Lagerung bei 30°C noch eine Enzymaktivität von etwa 80% gefunden, während in einem Vergleichsansatz ohne Kaliumsalz keine enzymatische Restaktivität mehr nachweisbar war. Außerdem konnte selbst bei einer Lagerung des erfindungsgemäßen Wasch- und Reinigungsmittels bei 0°C keine wesentliche Trübung oder Ausflockung festgestellt werden. Die erfindungsgemäßen Wasch- und Reinigungsmittel weisen daher eine gute Kältestabilität auf.

Gegenstand der vorliegenden Erfindung ist somit ein flüssiges Wasch- oder Reinigungsmittel, insbesondere ein Handgeschirrspülmittel, enthaltend Kaliumacetat und mindestens ein Enzym. Der Anteil der Kaliumionen an der Gesamtzusammensetzung beträgt 0,02 bis 0,5 Gew.-%.

In einer besonders bevorzugten Ausführungsform ist der Anteil der Kaliumionen an der Gesamtzusammensetzung 0,04 Gew.-%.

Unter flüssigen Reinigungsmitteln werden im Rahmen der vorliegenden Erfindung solche verstanden, die unter normalen Anwendungsbedingungen fließfähig sind und deren Viskositäten in einem breiten Rahmen variieren können. Zu den flüssigen Zubereitungen zählen auch gelförmige oder pastöse Mittel, welche gegebenenfalls zusätzliche aus dem Stand der Technik bekannte Verdickungsmittel aufweisen können. In einer weiter bevorzugten Ausführungsform der Erfindung beruhen die flüssigen Mittel auf wässeriger Basis.

Ein Handgeschirrspülmittel (synonym: manuelles Geschirrspülmittel) ist im Kontext der vorliegenden Erfindung ein flüssiges Wasch- oder Reinigungsmittel, das an die Verwendung zum manuellen Spülen von Geschirr besonders angepasst ist. Handgeschirrspülmittel sind demnach besonders dazu geeignet, Schmutz von harten Oberflächen zu lösen, weisen ein gutes Schaumvermögen und zudem eine besondere Hautverträglichkeit auf.

Das Handgeschirrspülmittel nach der vorliegenden Erfindung ist bevorzugt dadurch gekennzeichnet, dass es ein Schaumvermögen von mindestens 250 mL, gemessen nach der DIN-Methode 53 902, teil 2 (Ross-Miles-Test), vorzugsweise von mindestens 300 mL aufweist. Dieses vorteilhafte Schaumverhalten ist typischerweise darauf zurückzuführen, dass das Handgeschirrspülmittel vorzugsweise mindestens 5% Gew.-% eines anionischen Tensids, bezogen auf das gesamte Handgeschirrspülmittel, enthält. Der Gesamtgehalt an Tensid kann allerdings deutlich darüber liegen (siehe unten).

Um eine gute Hautverträglichkeit zu gewährleisten, liegt der pH Wert eines Handgeschirrspülmittels vorzugsweise im Bereich von (jeweils einschließlich) 4 bis 10. Bevorzugt sind pH Werte zwischen pH 5 und pH 9 (jeweils einschließlich), insbesondere bevorzugt ist ein pH Wert von 5,5 bis 8,5 (jeweils einschließlich). Vorzugsweise enthalten Handgeschirrspülmittel außerdem keine Bleichmittel wie z.B. Natriumpercarbonat und Natriumborat. Die Hautverträglichkeit lässt sich als Reizpotential nach der OECD-Methode No. 404 bestimmen. Es beträgt vorzugsweise höchstens 65%.Die Erfindung betrifft somit in einer besonders bevorzugten Ausführungsform ein flüssiges Wasch- oder Reinigungsmittel, das einen Mindestgehalt von 5 Gew.% eines anionischen Tensids, bezogen auf die gesamte Zusammensetzung, und einen pH Wert von ≤ 9 aufweist.

Ein erfindungsgemäßes Mittel enthält mindestens ein Enzym, vorzugsweise ein Enzym aus der Gruppe der bekannten, in Wasch- oder Reinigungsmitteln üblicherweise eingesetzten Enzyme. In einer bevorzugten Ausführungsform der Erfindung enthält das erfindungsgemäße Mittel eine Amylase und/oder eine Protease; dabei ist der Gehalt des Mittels an einer Amylase, gegebenenfalls in Kombination mit einer Protease, insbesondere bevorzugt. Dementsprechend bevorzugt ist die Verwendung von Kaliumsalzen, insbesondere Kaliumacetat, zur Erhöhung der Stabilität von Amylasen und/oder Proteasen, insbesondere in flüssigen Reinigungsmitteln, vor allem wässerigen Handgeschirrspülmitteln.

Für Amylasen können synonyme Begriffe verwendet werden, beispielsweise 1,4-alpha-D-Glucan-Glucanohydrolase oder Glycogenase. Erfindungsgemäß konfektionierbare Amylasen sind vorzugsweise α-Amylasen. Entscheidend dafür, ob ein Enzym eine α-Amylase im Sinne der Erfindung ist, ist deren Fähigkeit zur Hydrolyse von a(1-4)-Glykosidbindungen in der Amylose der Stärke.

Erfindungsgemäß konfektionierbare Amylasen sind beispielsweise die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyl®ultra, von dem Unternehmen Danisco/Genencor unter dem Namen Purastar®OxAm und von dem Unternehmen Daiwa Seiko Inc., Tokyo, Japan, als Keistase® erhältlich. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Des Weiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird.

Besonders geeignet für den Einsatz in erfindungsgemäßen Mitteln sind α-Amylase-Varianten der α-Amylase AA560 gemäß SEQ ID NO. 1. Folgende Varianten sind besonders vorteilhaft:
(a) α-Amylase-Variante, die gegenüber der α-Amylase AA560 gemäß SEQ ID NO. 1 eine, zwei, drei, vier, fünf oder sechs der folgenden Sequenzveränderungen in der Zählung der α-Amylase AA560 aufweist: R118K, D183* (Deletion), G184* (Deletion), N195F, R320K, R458K. Besonders bevorzugt weist die α-Amylase-Variante alle sechs der genannten Sequenzveränderungen auf.
(b) α-Amylase-Variante, die gegenüber der α-Amylase AA560 gemäß SEQ ID NO. 1 folgende Sequenzveränderungen aufweist (in der Zählung der α-Amylase AA560):
   (1) M9L / M202I,
   (2) M9L / M202I / M323T,
   (3) M9L / M202I / M323T / M382Y,
   (4) M9L / M202I / Y295F / A339S,
   (5) M9L / M202I / Y295F,
   (6) M9L / M202I / A339S,
   (7) M9L / M202I / Y295F / A339S,
   (8) M9L / M202I / Y295F / A339S / E345R,
   (9) M9L / G149A / M202I / Y295F / A339S / E345R,
   (10) M9L / M202L,
   (11) M9L / M202L / M323T,
   (12) M9L / M202L / M323T / M382Y,
   (13) M9L / M202L / Y295F / A339S,
   (14) M9L / M202L / Y295F,
   (15) M9L / M202L / A339S,
   (16) M9L / M202L / Y295F / A339S,
   (17) M9L / M202L / Y295F / A339S, E345R,
   (18) M9L / G149A / M202L / Y295F / A339S / E345R,
   (19) M9L / M202T,
   (20) M9L / M202T / M323T,
   (21) M9L / M202T / M323T / M382Y,
   (22) M9L / M202T / Y295F / A339S,
   (23) M9L / M202T / Y295F,
   (24) M9L / M202T / A339S,
   (25) M9L / M202T / Y295F / A339S,
   (26) M9L / M202T / Y295F / A339S / E345R,
   (27) M9L / G149A / M202T / Y295F / A339S / E345R,
   (28) M9L / G149A / M202I / V214T / Y295F / N299Y / M323T / A339S / E345R,
   (29) M9L / G149A / M202L / V214I / Y295F / M323T / A339S / E345R / M382Y,
   (30) M9L / G149A / G182T / G186A / M202I / V214I / Y295F / N299Y / M323T / A339S,
   (31) M9L / G149A / G182T / G186A / M202L / T257I / Y295F / N299Y / M323T / A339S / E345R,
   (32) M9L / G149A / M202L / V214T / Y295F / N299Y / M323T / A339S / E345R,
   (33) M9L / G149A / M202I / V214I / Y295F / M323T / A339S / E345R / M382Y,
   (34) M9L / G149A / G182T / G186A / M202L / V214I / Y295F / N299Y / M323T / A339S,
   (35) M9L / G149A / G182T / G186A / M202I / T257I / Y295F / N299Y / M323T / A339S / E345R,
   (36) M9L / G149A / M202I / V214T / Y295F / N299Y / M323T / A339S / E345R / N471E,
   (37) M9L / G149A / M202L / V214I / Y295F / M323T / A339S / E345R / M382Y / N471E,
   (38) M9L / G149A / G182T / G186A / M202I / V214I / Y295F / N299Y / M323T / A339S / N471E,
   (39) M9L / G149A / G182T / G186A / M202L / T257I / Y295F / N299Y / M323T / A339S / E345R / N471E,
   (40) M202L / M105F / M208F,
   (41) G133E / M202L / Q361E,
   (42) G133E / M202L / R444E,
   (43) M202L / Y295F,
   (44) M202L / A339S,
   (45) M202L / M323T,
   (46) M202L / M323T / M309L,
   (47) M202L / M323T / M430I,
   (48) M202L / V214T / R444Y,
   (49) M202L / N283D / Q361E,
   (50) M202L / M382Y / K383R,
   (51) M202L / K446R / N484Q,
   (52) M202I / Y295F,
   (53) M202I / A339S,
   (54) M202I / M105F / M208F,
   (55) G133E / M202I / Q361E,
   (56) G133E / M202I / R444E,
   (57) M202I / M323T,
   (58) M202I / M323T / M309L,
   (59) M202I / M323T / M430I,
   (60) M202I / V214T / R444Y,
   (61) M202I / N283D / Q361E,
   (62) M202I / M382Y / K383R,
   (63) M202I / K446R / N484Q,
   (64) M202V / M105F / M208F,
   (65) G133E / M202V / Q361E,
   (66) G133E / M202V / R444E,
   (67) M202V / M323T,
   (68) M202V / M323T / M309L,
   (69) M202V / M323T / M430I,
   (70) M202V / M323T / M9L,
   (71) M202V / V214T / R444Y,
   (72) M202V / N283D / Q361E,
   (73) M202V / M382Y / K383R,
   (74) M202V / K446R / N484Q,
   (75) M202T / M105F / M208F,
   (76) G133E / M202T / Q361E,
   (77) G133E / M202T / R444E,
   (78) M202T / Y295F,
   (79) M202T / A339S,
   (80) M202T / M323T,
   (81) M202T / M323T / M309L,
   (82) M202T / M323T / M430I,
   (83) M202T / M323T / M9L,
   (84) M202T / V214T / R444Y,
   (85) M202T / N283D / Q361E,
   (86) M202T / A339S,
   (87) M202T / Y295F
   (88) M202T / N299F,Y,
   (89) M202T / M382Y / K383R oder
   (90) M202T / K446R / N484Q
   Hierunter ganz besonders bevorzugt sind folgende α-Amylase-Varianten:
   (10) M9L / M202L,
   (28) M9L / G149A / M202I / V214T / Y295F / N299Y / M323T / A339S / E345R,
   (31) M9L / G149A / G182T / G186A / M202L / T257I / Y295F / N299Y / M323T / A339S / E345R,
   (35) M9L / G149A / G182T / G186A / M202I / T257I / Y295F / N299Y / M323T /
   (38) M9L / G149A / G182T / G186A / M202I / V214I / Y295F / N299Y / M323T /
   (39) M9L / G149A / G182T / G186A / M202L / T257I / Y295F / N299Y / M323T / A339S / E345R / N471E,
   (45) M202L / M323T,
   (46) M202L / M323T / M309L,
   (62) M202I / M382Y / K383R,
   (68) M202V / M323T / M309L,
   (73) M202V / M382Y / K383R
   (82) M202T / M323T / M430I oder
   (84) M202T / V214T / R444Y
(c) α-Amylase-Variante gemäß (b), die zusätzlich alle sechs unter (a) genannten Sequenzveränderungen aufweist, hierunter ganz besonders bevorzugt Variante 31 mit den sechs unter (a) genannten Sequenzveränderungen.

Erfindungsgemäß ganz besonders bevorzugt ist die vorstehend unter (a) genannte α-Amylase-Variante sowie die unter (c) genannte α-Amylase-Variante 31 mit den sechs unter (a) genannten Sequenzveränderungen.

Erfindungsgemäß bevorzugte flüssige Reinigungsmittel und Handgeschirrspülmittel enthalten, bezogen auf ihr Gesamtgewicht, zwischen 0,001 und 5,0 Gew.-%, vorzugsweise zwischen 0,01 und 4,0 Gew.-% und insbesondere zwischen 0,05 und 3,0 Gew.-% Amylase-Zubereitungen. Besonders bevorzugt werden flüssige Handgeschirrspülmittel, die bezogen auf ihr Gesamtgewicht zwischen 0,07 und 2,0 Gew.-% Amylase-Zubereitungen enthalten.

Erfindungsgemäß bevorzugte flüssige Reinigungsmittel und Handgeschirrspülmittel enthalten, bezogen auf ihr Gesamtgewicht, zwischen 0,002 und 7,0 Gew.-%, vorzugsweise zwischen 0,02 und 6,0 Gew.-% und insbesondere zwischen 0,1 und 5,0 Gew.-% Protease-Zubereitungen. Besonders bevorzugt werden Handgeschirrspülmittel, die bezogen auf ihr Gesamtgewicht zwischen 0,2 und 4,0 Gew.-% Protease-Zubereitungen enthalten.

In einer bevorzugten Ausführungsform enthält das Mittel dagegen als Enzymkomponente ausschließlich eine oder mehrere Amylasen und keine Protease.

Aus den bereits oben genannten Gründen werden wasch- oder reinigungsaktive Amylasen und Proteasen in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, welche vorteilhafterweise möglichst konzentriert, wasserarm und/oder mit Stabilisatoren oder weiteren Hilfsmitteln versetzt sind.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus den vorherigen Ausführungen ersichtlich, bildet das Enzym-Protein nur einen Bruchteil des Gesamtgewichts üblicher Enzym-Zubereitungen. Erfindungsgemäß bevorzugt eingesetzte Protease- und/oder Amylase-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 10 Gew.-% des Enzymproteins, jeweils bezogen auf die Enzym-Zubereitung.

Neben der Amylase und/oder Protease können die erfindungsgemäßen Mittel noch ein oder mehrere weitere wasch- oder reinigungsaktive Enzyme enthalten. Geeignet sind dabei insbesondere solche aus der Klasse der Hydrolasen wie der (Poly)Esterasen, Lipasen, Glykosylhydrolasen, Hemicellulasen, zu denen insbesondere Mannanasen, Xanthanlyasen, Pektinlyasen (=Pektinasen), Pektinesterasen, Pektatlyasen, Xyloglucanasen (=Xylanasen), Pullulanasen und β-Glucanasen gezählt werden, Cutinasen, β-Glucanasen, Oxidasen, Peroxidasen, Mannanasen, Perhydrolasen, Oxireduktasen und/oder Laccasen. Für weitere mögliche Enzyme und Enzym-Zubereitungen wird auf den einschlägigen Stand der Technik zu Wasch- oder Reinigungsmitteln verwiesen. Der Gewichtsanteil aller wasch- oder reinigungsaktiven Enzym-Zubereitungen am Gesamtgewicht des erfindungsgemäßen Mittels beträgt vorzugsweise zwischen 0,5 und 15 Gew.-%, bevorzugt zwischen 0,5 und 12 Gew.-%, besonders bevorzugt zwischen 0,6 und 10 Gew.-% und insbesondere zwischen 0,7 und 8 Gew.-%.

Mit besonderem Vorteil werden manuelle Geschirrspülmittel bereitgestellt, welche als Enzyme eine Kombination aus Amylase und Protease sowie gegebenenfalls weitere Enzyme enthalten. Der Gewichtsanteil aller wasch- oder reinigungsaktiven Enzym-Zubereitungen am Gesamtgewicht des erfindungsgemäßen manuellen Geschirrspülmittels beträgt vorzugsweise zwischen 0,5 und 5 Gew.-%, bevorzugt zwischen 0,7 und 3 Gew.-%.

Die erfindungsgemäßen Mittel enthalten als weiteren Bestandteil üblicherweise Tenside, vor allem anionische Tenside, nichtionische Tenside, amphotere Tenside, vorzugsweise Betaine sowie ggf. kationische Tenside. Die Gesamtmenge der Tenside in den erfindungsgemäßen Mitteln kann in einem breiten Rahmen variieren und beispielsweise 5 bis 70 Gew.-%, vorzugsweise 7 bis 55 Gew.-% und insbesondere 10 bis 50 Gew.-% betragen. Es ist insbesondere bevorzugt, dass der Anteil von anionischen Tensiden ≥ 5 Gew.-% an dem Gesamtgewicht der erfindungsgemäßen Zusammensetzung beträgt.

Vorzugsweise ist neben dem anionischen Tensid noch mindestens ein amphoteres Tensid vorhanden, vorzugsweise ein Betain. In einer ganz besonders bevorzugten Ausführungsform besteht der Tensidanteil des erfindungsgemäßen Mittels aus einem Tensid ausgewählt aus der Gruppe der anionischen Tenside und einem Tensid ausgewählt aus der Gruppe der amphoteren Tenside.

Die anionischen Tenside werden üblicherweise als Alkalimetall-, Erdalkalimetall- und/oder Mono-, Di- bzw. Trialkanolammoniumsalz und/oder aber auch in Form ihrer mit dem entsprechenden Alkalimetallhydroxid, Erdalkalimetallhydroxid und/oder Mono-, Di- bzw. Trialkanolamin in situ zu neutralisierenden korrespondierenden Säure eingesetzt. Bevorzugt sind hierbei als Alkalimetalle Natrium, als Erdalkalimetalle Calcium und insbesondere Magnesium, sowie als Alkanolamine Mono-, Di- oder Triethanolamin. Besonders bevorzugt sind die Natriumsalze. Selbst wenn in manuellen Geschirrspülmitteln Aniontenside in Form ihrer Calciumsalze eingesetzt werden, enthalten die Mittel erfindungsgemäß zusätzlich Calciumlactat, vorzugsweise in den oben angegebenen Mengen.

Zu den insbesondere in manuellen Geschirrspülmitteln bevorzugt eingesetzten Aniontensiden zählen vor allem Alkylethersulfate und Alkylsulfonate.

Alkylethersulfate (Fettalkoholethersulfate, INCI Alkyl Ether Sulfates) sind Produkte von Sulfatierreaktionen an alkoxylierten Alkoholen. Dabei versteht der Fachmann allgemein unter alkoxylierten Alkoholen die Reaktionsprodukte von Alkylenoxid, bevorzugt Ethylenoxid, mit Alkoholen, im Sinne der vorliegenden Erfindung bevorzugt mit längerkettigen Alkoholen, d.h. mit aliphatischen geradkettigen oder ein oder mehrfach verzweigten, acyclischen oder cyclischen, gesättigten oder ein oder mehrfach ungesättigten, vorzugsweise geradkettigen, acyclischen, gesättigten, Alkoholen mit 6 bis 22, vorzugsweise 8 bis 18, insbesondere 10 bis 16 und besonders bevorzugt 12 bis 14 Kohlenstoffatomen. In der Regel entsteht aus n Molen Ethylenoxid und einem Mol Alkohol, abhängig von den Reaktionsbedingungen, ein komplexes Gemisch von Additionsprodukten unterschiedlicher Ethoxylierungsgrade (n = 1 bis 30, vorzugsweise 1 bis 20, insbesondere 1 bis 10, besonders bevorzugt 2 bis 4). Eine weitere Ausführungsform der Alkoxylierung besteht im Einsatz von Gemischen der Alkylenoxide, bevorzugt des Gemisches von Ethylenoxid und Propylenoxid. Ganz besonders bevorzugt im Sinne der vorliegenden Erfindung sind niederethoxylierte Fettalkohole mit 1 bis 4 Ethylenoxideinheiten (EO), insbesondere 1 bis 2 EO, beispielsweise 2 EO, wie Na-C₁₂₋₁₄-Fettalkohol+2EO-sulfat.

Das erfindungsgemäße Mittel, insbesondere ein manuelles Geschirrspülmittel, enthält in einer bevorzugten Ausführungsform ein oder mehrere Alkylethersulfate in einer Menge von 5 bis 40 Gew.-%, vorzugsweise 6,5 bis 35 Gew.-%, insbesondere 8 bis 30 Gew.-%.

Die Alkylsulfonate (INCI Sulfonic Acids) weisen üblicherweise einen aliphatischen geradkettigen oder ein- oder mehrfach verzweigten, acyclischen oder cyclischen, gesättigten oder ein- oder mehrfach ungesättigten, vorzugsweise verzweigten, acyclischen, gesättigten, Alkylrest mit 6 bis 22, vorzugsweise 9 bis 20, insbesondere 11 bis 18 und besonders bevorzugt 14 bis 17 Kohlenstoffatomen auf.

Geeignete Alkylsulfonate sind dementsprechend die gesättigten Alkansulfonate, die ungesättigten Olefinsulfonate und die - sich formal von den auch den Alkylethersulfaten zugrunde liegenden alkoxylierten Alkoholen ableitenden - Ethersulfonate, bei denen man endständige Ethersulfonate (n-Ethersulfonate) mit an die Polyether-Kette gebundener Sulfonat-Funktion und innenständige Ethersulfonate (i-Ethersulfonate) mit mit dem Alkylrest verknüpfter Sulfonat-Funktion unterscheidet.

Erfindungsgemäß bevorzugt sind die Alkansulfonate, insbesondere Alkansulfonate mit einem verzweigten, vorzugsweise sekundären, Alkylrest, beispielsweise das sekundäre Alkansulfonat sek. Na-C₁₃₋₁₇-Alkansulfonat (INCI Sodium C14-17 Alkyl Sec Sulfonate).

Ein bevorzugtes manuelles Geschirrspülmittel enthält ein oder mehrere sekundäre Alkylsulfonate in einer Menge von 1 bis 15 Gew.-%, vorzugsweise 3 bis 10 Gew.-%, insbesondere 4 bis 8 Gew.-%.

Weitere mögliche einsetzbare Aniontenside sind dem Fachmann aus dem einschlägigen Stand der Technik zu Wasch- oder Reinigungsmitteln bekannt. Hierzu zählen insbesondere aliphatische Sulfate wie Fettalkoholsulfate, Monoglyceridsulfate sowie Estersulfonate (Sulfofettsäureester), Ligninsulfonate, Alkylbenzolsulfonate, Fettsäurecyanamide, anionische Sulfobernsteinsäuretenside, Fettsäureisethionate, Acylaminoalkansulfonate (Fettsäuretauride), Fettsäuresarcosinate, Ethercarbonsäuren und Alkyl(ether)phosphate.

Geeignete weitere anionische Tenside sind auch anionische Gemini-Tenside mit einer Diphenyloxid-Grundstruktur, 2 Sulfonatgruppen und einem Alkylrest an einem oder beiden Benzolringen gemäß der Formel ⁻O₃S(C₆H₃R)O(C₆H₃R')SO₃⁻, in der R für einen Alkylrest mit beispielsweise 6, 10, 12 oder 16 Kohlenstoffatomen und R' für R oder H steht (Dowfax® Dry Hydrotrope Powder mit C₁₆-Alkylrest(en); INCI Sodium Hexyldiphenyl Ether Sulfonate, Disodium Decyl Phenyl Ether Disulfonate, Disodium Lauryl Phenyl Ether Disulfonate, Disodium Cetyl Phenyl Ether Disulfonate).

Besonders bevorzugte weitere anionische Tenside sind die anionischen Sulfobernsteinsäuretenside Sulfosuccinate, Sulfosuccinamate und Sulfosuccinamide, insbesondere Sulfosuccinate und Sulfosuccinamate, äußerst bevorzugt Sulfosuccinate. Bei den Sulfosuccinaten handelt es sich um die Salze der Mono- und Diester der Sulfobernsteinsäure HOOCCH(SO₃H)CH₂COOH, während man unter den Sulfosuccinamaten die Salze der Monoamide der Sulfobernsteinsäure und unter den Sulfosuccinamiden die Salze der Diamide der Sulfobernsteinsäure versteht. Bei den Salzen handelt es sich bevorzugt um Alkalimetallsalze, Ammoniumsalze sowie Mono-, Di- bzw. Trialkanolammoniumsalze, beispielsweise Mono-, Di- bzw. Triethanolammoniumsalze, insbesondere um Lithium-, Natrium- oder Ammoniumsalze, besonders bevorzugt Natrium- oder Ammoniumsalze, äußerst bevorzugt Natriumsalze.

In den Sulfosuccinaten ist eine bzw. sind beide Carboxylgruppen der Sulfobernsteinsäure vorzugsweise mit einem bzw. zwei gleichen oder verschiedenen unverzweigten oder verzweigten, gesättigten oder ungesättigten, acyclischen oder cyclischen, optional alkoxylierten Alkoholen mit 4 bis 22, vorzugsweise 6 bis 20, insbesondere 8 bis 18, besonders bevorzugt 10 bis 16, äußerst bevorzugt 12 bis 14 Kohlenstoffatomen verestert. Besonders bevorzugt sind die Ester unverzweigter und/oder gesättigter und/oder acyclischer und/oder alkoxylierter Alkohole, insbesondere unverzweigter, gesättigter Fettalkohole und/oder unverzweigter, gesättigter, mit Ethylen- und/oder Propylenoxid, vorzugsweise Ethylenoxid, alkoxylierter Fettalkohole mit einem Alkoxylierungsgrad von 1 bis 20, vorzugsweise 1 bis 15, insbesondere 1 bis 10, besonders bevorzugt 1 bis 6, äußerst bevorzugt 1 bis 4. Die Monoester werden im Rahmen der vorliegenden Erfindung gegenüber den Diestern bevorzugt. Ein besonders bevorzugtes Sulfosuccinat ist Sulfobernsteinsäurelaurylpolyglykolester-di-Natrium-Salz (Lauryl-EO-sulfosuccinat, Di-Na-Salz; INCI Disodium Laureth Sulfosuccinate), das beispielsweise als Tego® Sulfosuccinat F 30 (Goldschmidt) mit einem Sulfosuccinatgehalt von 30 Gew.-% kommerziell erhältlich ist.

In den Sulfosuccinamaten bzw. Sulfosuccinamiden bildet eine bzw. bilden beide Carboxylgruppen der Sulfobernsteinsäure vorzugsweise mit einem primären oder sekundären Amin, das einen oder zwei gleiche oder verschiedene, unverzweigte oder verzweigte, gesättigte oder ungesättigte, acyclische oder cyclische, optional alkoxylierte Alkylreste mit 4 bis 22, vorzugsweise 6 bis 20, insbesondere 8 bis 18, besonders bevorzugt 10 bis 16, äußerst bevorzugt 12 bis 14 Kohlenstoffatomen trägt, ein Carbonsäureamid. Besonders bevorzugt sind unverzweigte und/oder gesättigte und/oder acyclische Alkylreste, insbesondere unverzweigte, gesättigte Fettalkylreste.

Weiterhin geeignet sind beispielsweise die folgenden gemäß INCI bezeichneten Sulfosuccinate und Sulfosuccinamate, die im International Cosmetic Ingredient Dictionary and Handbook näher beschrieben sind: Ammonium Dinonyl Sulfosuccinate, Ammonium Lauryl Sulfosuccinate, Diammonium Dimethicone Copolyol Sulfosuccinate, Diammonium Lauramido-MEA Sulfosuccinate, Diammonium Lauryl Sulfosuccinate, Diammonium Oleamido PEG-2 Sulfosuccinate, Diamyl Sodium Sulfosuccinate, Dicapryl Sodium Sulfosuccinate, Dicyclohexyl Sodium Sulfosuccinate, Diheptyl Sodium Sulfosuccinate, Dihexyl Sodium Sulfosuccinate, Diisobutyl Sodium Sulfosuccinate, Dioctyl Sodium Sulfosuccinate, Disodium Cetearyl Sulfosuccinate, Disodium Cocamido MEA-Sulfo-succinate, Disodium Cocamido MIPA-Sulfosuccinate, Disodium Cocamido PEG-3 Sulfosuccinate, Disodium Coco-Glucoside Sulfosuccinate, Disodium Cocoyl Butyl Gluceth-10 Sulfosuccinate, Disodium C12-15 Pareth Sulfosuccinate, Disodium Deceth-5 Sulfosuccinate, Disodium Deceth-6 Sulfosuccinate, Disodium Dihydroxyethyl Sulfosuccinylundecylenate, Disodium Dimethicone Copolyol Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Laneth-5 Sulfosuccinate, Disodium Lauramido MEA-Sulfosuccinate, Disodium Lauramido PEG-2 Sulfosuccinate, Disodium Lauramido PEG-5 Sulfosuccinate, Disodium Laureth-6 Sulfosuccinate, Disodium Laureth-9 Sulfosuccinate, Disodium Laureth-12 Sulfosuccinate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleth-3 Sulfosuccinate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfo-succinate, Disodium PEG-5 Laurylcitrate Sulfosuccinate, Disodium PEG-8 Palm Glycerides Sulfosuccinate, Disodium Ricinoleamido MEA-Sulfosuccinate, Disodium Sitostereth-14 Sulfosuccinate, Disodium Stearamido MEA-Sulfosuccinate, Disodium Stearyl Sulfosuccinamate, Disodium Stearyl Sulfosuccinate, Disodium Tallamido MEA-Sulfosuccinate, Disodium Tallowamido MEA-Sulfosuccinate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Wheat Germamido MEA-Sulfosuccinate, Disodium Wheat Germamido PEG-2 Sulfosuccinate, Di-TEA-Oleamido PEG-2 Sulfosuccinate, Ditridecyl Sodium Sulfosuccinate, Sodium Bisglycol Ricinosulfosuccinate, Sodium/MEA Laureth-2 Sulfosuccinate und Tetrasodium Dicarboxyethyl Stearyl Sulfosuccinamate. Noch ein weiteres geeignetes Sulfosuccinamat ist Dinatrium-C₁₆₋₁₈-alkoxypropylen-sulfosuccinamat.

Bevorzugte anionische Sulfobernsteinsäuretenside sind Imidosuccinat, Mono-Na-sulfobernsteinsäure-di-isobutylester (Monawet® MB 45), Mono-Na-sulfobernsteinsäure-di-octylester (Monawet® MO-84 R2W, Rewopol® SB DO 75), Mono-Na-sulfobernsteinsäure-di-tridecylester (Monawet® MT 70), Fettalkoholpolyglykolsulfosuccinat-Na-NH₄-Salz (Sulfosuccinat S-2), Di-Na-sulfobernstein-säuremono-C_{12/14}-3EO-ester (Texapon® SB-3), Natruimsulfobernsteinsäurediisooctylester (Texin® DOS 75) und Di-Na-Sulfobernsteinsäure-mono-C_{12/18}-ester (Texin® 128-P), insbesondere der mit der erfindungsgemäßen ternären Tensidkombination hinsichtlich des Ablauf- und/oder Trocknungsverhaltens synergistisch zusammenwirkende Mono-Na-sulfobernsteinsäure-di-octylester.

In einer besonderen Ausführungsform enthält das erfindungsgemäße Mittel als anionische Sulfobernsteinsäuretenside ein oder mehrere Sulfosuccinate, Sulfosuccinamate und/oder Sulfosuccinamide, vorzugsweise Sulfosuccinate und/oder Sulfosuccinamate, insbesondere Sulfosuccinate, in einer Menge von üblicherweise 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

Zu den Amphotensiden (amphoteren Tensiden, zwitterionischen Tensiden), die erfindungsgemäß eingesetzt werden können, zählen Alkylamidoalkylamine, alkylsubstituierte Aminosäuren, acylierte Aminosäuren bzw. Biotenside, von denen die Betaine im Rahmen der erfindungsgemäßen Lehre bevorzugt werden.

Geeignete Betaine, welche vor allem in manuellen Geschirrspülmitteln Einsatz finden, sind die Alkylbetaine, die Alkylamidobetaine, die Imidazoliniumbetaine, die Sulfobetaine (INCI Sultaines) sowie die Phosphobetaine und genügen vorzugsweise Formel I,

R¹-[CO-X-(CH₂)ₙ]ₓ-N⁺(R²)(R³)-(CH₂)ₘ-[CH(OH)-CH₂]_{y}-Y⁻ (I)

in der
- R¹: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- X: NH, NR⁴ mit dem C₁₋₄-Alkylrest R⁴, O oder S,
- n: eine Zahl von 1 bis 10, vorzugsweise 2 bis 5, insbesondere 3,
- x: 0 oder 1, vorzugsweise 1,
- R², R³: unabhängig voneinander ein C₁₋₄-Alkylrest, ggf. hydroxysubstituiert wie z.B. ein Hydroxyethylrest, insbesondere aber ein Methylrest,
- m: eine Zahl von 1 bis 4, insbesondere 1, 2 oder 3,
- y: 0 oder 1 und
- Y: COO, SO₃, OPO(OR⁵)O oder P(O)(OR⁵)O, wobei R⁵ ein Wasserstoffatom H oder ein C₁₋₄-Alkylrest ist.

Die Alkyl- und Alkylamidobetaine, Betaine der Formel I mit einer Carboxylatgruppe (Y⁻ = COO⁻), heißen auch Carbobetaine.

Bevorzugte Betaine sind die Alkylbetaine der Formel (la), die Alkylamidobetaine der Formel (Ib), die Sulfobetaine der Formel (Ic) und die Amidosulfobetaine der Formel (Id),

R¹-N⁺(CH₃)₂-CH₂COO⁻ (Ia)

R¹-CO-NH-(CH₂)₃-N⁺(CH₃)₂-CH₂COO⁻ (Ib)

R¹-N⁺(CH₃)₂-CH₂CH(OH)CH₂SO₃⁻ (Ic)

R¹-CO-NH-(CH₂)₃-N⁺(CH₃)₂-CH₂CH(OH)CH₂SO₃⁻ (Id)

in denen R¹ die gleiche Bedeutung wie in Formel I hat.

Besonders bevorzugte Betaine sind die Carbobetaine, insbesondere die Carbobetaine der Formel (la) und (Ib), äußerst bevorzugt die Alkylamidobetaine der Formel (Ib).

Beispiele geeigneter Betaine und Sulfobetaine sind die folgenden gemäß INCI benannten Verbindungen: Almondamidopropyl Betaine, Apricotamidopropyl Betaine, Avocadamidopropyl Betaine, Babassuamidopropyl Betaine, Behenamidopropyl Betaine, Behenyl Betaine, Betaine, Canolamidopropyl Betaine, Capryl/Capramidopropyl Betaine, Carnitine, Cetyl Betaine, Cocamidoethyl Betaine, Cocamidopropyl Betaine, Cocamidopropyl Hydroxysultaine, Coco-Betaine, Coco-Hydroxysultaine, Coco/Oleamidopropyl Betaine, Coco-Sultaine, Decyl Betaine, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl PG-Betaine, Erucamidopropyl Hydroxysultaine, Hydrogenated Tallow Betaine, Isostearamidopropyl Betaine, Lauramidopropyl Betaine, Lauryl Betaine, Lauryl Hydroxysultaine, Lauryl Sultaine, Milkamidopropyl Betaine, Minkamidopropyl Betaine, Myristamidopropyl Betaine, Myristyl Betaine, Oleamidopropyl Betaine, Oleamidopropyl Hydroxysultaine, Oleyl Betaine, Olivamidopropyl Betaine, Palmamidopropyl Betaine, Palmitamidopropyl Betaine, Palmitoyl Carnitine, Palm Kernelamidopropyl Betaine, Polytetrafluoroethylene Acetoxypropyl Betaine, Ricinoleamidopropyl Betaine, Sesamidopropyl Betaine, Soyamidopropyl Betaine, Stearamidopropyl Betaine, Stearyl Betaine, Tallowamidopropyl Betaine, Tallowamidopropyl Hydroxysultaine, Tallow Betaine, Tallow Dihydroxyethyl Betaine, Undecylenamidopropyl Betaine und Wheat Germamidopropyl Betaine. Ein bevorzugtes Betain ist beispielsweise Cocamidopropyl Betaine (Cocoamidopropylbetain).

Das erfindungsgemäße Mittel enthält in einer bevorzugten Ausführungsform ein oder mehrere Betaine in einer Menge von 0,5 bis 15 Gew.-%, vorzugsweise 1 bis 10 Gew.-%, insbesondere 1 bis 8 Gew.-%.

In einer bevorzugten Ausführungsform sind im erfindungsgemäßen Mittel die folgenden Tenside a) Alkylethersulfat, b) sekundäres Alkansulfonat und c) Betain vorhanden, vorzugsweise in einem Verhältnis a): b): c) von 5:2:1 bis 3:1:1.

In einer anderen bevorzugten Ausführungsform sind im erfindungsgemäßen Mittel die folgenden Tenside a) Alkylethersulfat und b) Betain vorhanden, vorzugsweise in einem Verhältnis a) : b) von 5:1 bis 10:1, bevorzugt 6:1 bis 9:1, besonders bevorzugt ungefähr 7:1.

Die Alkylamidoalkylamine (INCI Alkylamido Alkylamines) sind Amphotenside der Formel (III),

R⁹-CO-NR¹⁰-(CH₂)ᵢ-N(R¹¹)-(CH₂CH₂O)ⱼ-CH₂)ₖ-[CH(OH)]ₗ-CH₂-Z-OM (III)

in der
- R⁹: ein gesättiger oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁰: ein Wasserstoffatom H oder ein C₁₋₄-Alkylrest, vorzugsweise H,
- i: eine Zahl von 1 bis 10, vorzugsweise 2 bis 5, insbesondere 2 oder 3,
- R¹¹: ein Wasserstoffatom H oder CH₂COOM (zu M s.u.),
- j: eine Zahl von 1 bis 4, vorzugsweise 1 oder 2, insbesondere 1,
- k: eine Zahl von 0 bis 4, vorzugsweise 0 oder 1,
- I: 0 oder 1, wobei k = 1 ist, wenn I = 1 ist,
- Z: CO, SO₂, OPO(OR¹²) oder P(O)(OR¹²), wobei R¹² ein C₁₋₄-Alkylrest oder M (s.u.) ist, und
- M: ein Wasserstoff, ein Alkalimetall, ein Erdalkalimetall oder ein protoniertes Alkanolamin, z.B. protoniertes Mono-, Di- oder Triethanolamin, ist.

Bevorzugte Vertreter genügen den Formeln IIIa bis IIId,

R⁹-CO-NH-(CH₂)₂-N(R¹¹)-CH₂CH₂O-CH₂-COOM (IIIa)

R⁹-CO-NH-(CH₂)₂-N(R¹¹)-CH₂CH₂O-CH₂CH₂-COOM (IIIb)

R⁹-CO-NH-(CH₂)₂-N(R¹¹)-CH₂CH₂O-CH₂CH(OH)CH₂-SO₃M (IIIc)

R⁹-CO-NH-(CH₂)₂-N(R¹¹)-CH₂CH₂O-CH₂CH(OH)CH₂-OPO₃HM (IIId)

in denen R¹¹ und M die gleiche Bedeutung wie in Formel (III) haben.

Beispielhafte Alkylamidoalkylamine sind die folgenden gemäß INCI benannten Verbindungen: Cocoamphodipropionic Acid, Cocobetainamido Amphopropionate, DEA-Cocoamphodipropionate, Disodium Caproamphodiacetate, Disodium Caproamphodipropionate, Disodium Capryloamphodiacetate, Disodium Capryloamphodipropionate, Disodium Cocoamphocarboxyethylhydroxypropylsulfonate, Disodium Cocoamphodiacetate, Disodium Cocoamphodipropionate, Disodium Isostearoamphodiacetate, Disodium Isostearoamphodipropionate, Disodium Laureth-5 Carboxyamphodiacetate, Disodium Lauroamphodiacetate, Disodium Lauroamphodipropionate, Disodium Oleoamphodipropionate, Disodium PPG-2-lsodeceth-7 Carboxyamphodiacetate, Disodium Stearoamphodiacetate, Disodium Tallowamphodiacetate, Disodium Wheatgermamphodiacetate, Lauroamphodipropionic Acid, Quaternium-85, Sodium Caproamphoacetate, Sodium Caproamphohydroxypropylsulfonate, Sodium Caproamphopropionate, Sodium Capryloamphoacetate, Sodium Capryloamphohydroxypropylsulfonate, Sodium Capryloamphopropionate, Sodium Cocoamphoacetate, Sodium Cocoamphohydroxypropylsulfonate, Sodium Cocoamphopropionate, Sodium Cornamphopropionate, Sodium Isostearoamphoacetate, Sodium Isostearoamphopropionate, Sodium Lauroamphoacetate, Sodium Lauroamphohydroxypropylsulfonate, Sodium Lauroampho PG-Acetate Phosphate, Sodium Lauroamphopropionate, Sodium Myristoamphacetate, Sodium Oleoamphoacetate, Sodium Oleoamphohydroxypropylsulfonate, Sodium Oleoamphopropionate, Sodium Ricinoleoamphoacetate, Sodium Stearoamphoacetate, Sodium Stearoamphohydroxypropylsulfonate, Sodium Stearoamphopropionate, Sodium Tallamphopropionate, Sodium Tallowamphoacetate, Sodium Undecylenoamphoacetate, Sodium Undecylenoamphopropionate, Sodium Wheat Germamphoacetate und Trisodium Lauroampho PG-Acetate Chloride Phosphate.

Erfindungsgemäß bevorzugte alkylsubstituierte Aminosäuren (INCI Alkyl-Substituted Amino Acids) sind monoalkylsubstituierte Aminosäuren gemäß Formel (IV),

R¹³-NH-CH(R¹⁴)-(CH₂)ᵤ-COOM' (IV)

in der
- R¹³: ein gesättiger oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁴: ein Wasserstoffatom H oder ein C₁₋₄-Alkylrest, vorzugsweise H,
- u: eine Zahl von 0 bis 4, vorzugsweise 0 oder 1, insbesondere 1, und
- M': ein Wasserstoff, ein Alkalimetall, ein Erdalkalimetall oder ein protoniertes Alkanolamin, z.B. protoniertes Mono-, Di- oder Triethanolamin, ist,
alkylsubstituierte Iminosäuren gemäß Formel (V),

R¹⁵-N-[(CH₂)ᵥ-COOM"]₂ (V)

in der
- R¹⁵: ein gesättiger oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- v: eine Zahl von 1 bis 5, vorzugsweise 2 oder 3, insbesondere 2, und
- M": ein Wasserstoff, ein Alkalimetall, ein Erdalkalimetall oder ein protoniertes Alkanolamin, z.B. protoniertes Mono-, Di- oder Triethanolamin, wobei M" in den beiden Carboxygruppen die gleiche oder zwei verschiedene Bedeutungen haben kann, z.B. Wasserstoff und Natrium oder zweimal Natrium sein kann, ist,
und mono- oder dialkylsubstituierte natürliche Aminosäuren gemäß Formel (VI),

R¹⁶-N(R¹⁷)-CH(R¹⁸)-COOM'" (VI)

in der
- R¹⁶: ein gesättiger oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁷: ein Wasserstoffatom oder ein C₁₋₄-Alkylrest, ggf. hydroxy- oder aminsubstituiert, z.B. ein Methyl-, Ethyl-, Hydroxyethyl- oder Aminpropylrest,
- R¹⁸: den Rest einer der 20 natürlichen α-Aminosäuren H₂NCH(R¹⁸)COOH, und
- M'": ein Wasserstoff, ein Alkalimetall, ein Erdalkalimetall oder ein protoniertes Alkanolamin, z.B. protoniertes Mono-, Di- oder Triethanolamin, ist.

Besonders bevorzugte alkylsubstituierte Aminosäuren sind die Aminopropionate gemäß Formel (IVa),

R¹³-NH-CH₂CH₂COOM' (IVa)

in der R¹³ und M' die gleiche Bedeutung wie in Formel (IV) haben.

Beispielhafte alkylsubstituierte Aminosäuren sind die folgenden gemäß INCI benannten Verbindungen: Aminopropyl Laurylglutamine, Cocaminobutyric Acid, Cocaminopropionic Acid, DEA-Lauraminopropionate, Disodium Cocaminopropyl Iminodiacetate, Disodium Dicarboxyethyl Cocopropylenediamine, Disodium Lauriminodipropionate, Disodium Steariminodipropionate, Disodium Tallowiminodipropionate, Lauraminopropionic Acid, Lauryl Aminopropylglycine, Lauryl Diethylenediaminoglycine, Myristaminopropionic Acid, Sodium C12-15 Alkoxypropyl Iminodipropionate, Sodium Cocaminopropionate, Sodium Lauraminopropionate, Sodium Lauriminodipropionate, Sodium Lauroyl Methylaminopropionate, TEA-Lauraminopropionate und TEA-Myristaminopropionate.

Acylierte Aminosäuren sind Aminosäuren, insbesondere die 20 natürlichen α-Aminosäuren, die am Aminostickstoffatom den Acylrest R¹⁹CO einer gesättigten oder ungesättigen Fettsäure R¹⁹COOH tragen, wobei R¹⁹ ein gesättiger oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest ist. Die acylierten Aminosäuren können auch als Alkalimetallsalz, Erdalkalimetallsalz oder Alkanolammoniumsalz, z.B. Mono-, Di- oder Triethanolammoniumsalz, eingesetzt werden. Beispielhafte acylierte Aminosäuren sind die gemäß INCI unter Amino Acids zusammengefassten Acylderivate, z.B. Sodium Cocoyl Glutamate, Lauroyl Glutamic Acid, Capryloyl Glycine oder Myristoyl Methylalanine.

In einer besonderen Ausführungsform der Erfindung wird eine Kombination aus zwei oder mehr verschiedenen Amphotensiden, insbesondere eine binäre Amphotensidkombination eingesetzt.
Die Amphotensidkombination enthält vorzugsweise mindestens ein Betain, insbesondere mindestens ein Alkylamidobetain, besonders bevorzugt Cocoamidopropylbetain.

Weiterhin enthält die Amphotensidkombination vorzugsweise mindestens ein amphoteres Tensid aus der Gruppe umfassend Natriumcarboxyethylkokosphosphoethylimidazolin (Phosphoteric® TC-6), C_{8/10}-Amidopropylbetain (INCI Capryl/Capramidopropyl Betaine; Tego® Betaine 810), N-2-Hydroxyethyl-N-carboxymethyl-fettsäureamido-ethylamin-Na (Rewoteric® AMV) und N-Capryl/Caprin-amidoethyl-N-ethylether-propionat-Na (Rewoteric® AMVSF) sowie das Betain 3-(3-Cocoamido-propyl)-dimethylammonium-2-hydroxypropansulfonat (INCI Sultaine; Rewoteric® AM CAS) und das Alkylamidoalkylamin N-[N'(N"-2-Hydroxyethyl-N"-carboxyethylaminoethyl)-essigsäureamido]-N,N-dimethyl-N-cocos-ammoniumbetain (Rewoteric® QAM 50), insbesondere zusammen mit Cocoamidopropylbetain.

In einer weiteren besonderen Ausführungsform enthält das erfindungsgemäße Mittel ein oder mehrere Amphotenside in einer Menge von mehr als 8 Gew.-%. In noch einer weiteren besonderen Ausführungsform enthält das erfindungsgemäße Mittel ein oder mehrere Amphotenside in einer Menge von weniger als 2 Gew.-%.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Insbesondere bevorzugt enthält das Handgeschirrspülmittel einen C₁₂₋₁₈-Fettalkohol mit 7 EO oder einen C₁₃₋₁₅-Oxoalkohol mit 7 EO als nichtionisches Tensid.
Der Gehalt an nichtionischen Tensiden beträgt in dem Handgeschirrspülmittel bevorzugt 1 bis 30 Gew.-% und vorzugsweise 2 bis 25 Gew.-%, jeweils bezogen auf das gesamte Handgeschirrspülmittel.

Diese nichtionische Tenside weisen in Kombination mit einem Aminoxid eine gute Reinigungsleistung an Fett-verschmutzten harten Oberflächen, wie beispielsweise Geschirr, auf.

Nichtionische Tenside im Rahmen der Erfindung sind Alkoxylate, aber auch Alkylphenolpolyglykolether, endgruppenverschlossene Polyglykolether, Mischether und Hydroxymischether und Fettsäurepolyglykolester. Ebenfalls geeignet sind Blockpolymere aus Ethylenoxid und Propylenoxid sowie Fettsäurealkanolamide und Fettsäurepolyglykolether. Wichtige Klassen erfindungsgemäßer nichtionischer Tenside sind weiterhin die Aminoxide und die Zuckertenside, insbesondere die Alkylpolyglucoside.

Zu den erfindungsgemäß geeigneten Aminoxiden gehören Alkylaminoxide, insbesondere Alkyldimethylaminoxide, Alkylamidoaminoxide und Alkoxyalkylaminoxide. Bevorzugte Aminoxide genügen Formel II,

R⁶R⁷R⁸N⁺-O⁻ (II)

R⁶-[CO-NH-(CH₂)_{w}]_{z}-N⁺(R⁷)(R⁸)-O⁻ (II)

in der
- R⁶: ein gesättiger oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest, der in den Alkylamidoaminoxiden über eine Carbonylamidoalkylengruppe -CO-NH-(CH₂)_{z}- und in den Alkoxyalkylaminoxiden über eine Oxaalkylengruppe -O-(CH₂)_{z}- an das Stickstoffatom N gebunden ist, wobei z jeweils für eine Zahl von 1 bis 10, vorzugsweise 2 bis 5, insbesondere 3,
- R⁷, R⁸: unabhängig voneinander ein C₁₋₄-Alkylrest, ggf. hydroxysubstituiert wie z.B. ein Hydroxyethylrest, insbesondere ein Methylrest, ist.

Beispiele geeigneter Aminoxide sind die folgenden gemäß INCI benannten Verbindungen: Almondamidopropylamine Oxide, Babassuamidopropylamine Oxide, Behenamine Oxide, Cocamidopropyl Amine Oxide, Cocamidopropylamine Oxide, Cocamine Oxide, Coco-Morpholine Oxide, Decylamine Oxide, Decyltetradecylamine Oxide, Diaminopyrimidine Oxide, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Hydrogenated Palm Kernel Amine Oxide, Hydrogenated Tallowamine Oxide, Hydroxyethyl Hydroxypropyl C12-15 Alkoxypropylamine Oxide, Isostearamidopropylamine Oxide, Isostearamidopropyl Morpholine Oxide, Lauramidopropylamine Oxide, Lauramine Oxide, Methyl Morpholine Oxide, Milkamidopropyl Amine Oxide, Minkamidopropylamine Oxide, Myristamidopropylamine Oxide, Myristamine Oxide, Myristyl/Cetyl Amine Oxide, Oleamidopropylamine Oxide, Oleamine Oxide, Olivamidopropylamine Oxide, Palmitamidopropylamine Oxide, Palmitamine Oxide, PEG-3 Lauramine Oxide, Potassium Dihydroxyethyl Cocamine Oxide Phosphate, Potassium Trisphosphonomethylamine Oxide, Sesamidopropylamine Oxide, Soyamidopropylamine Oxide, Stearamidopropylamine Oxide, Stearamine Oxide, Tallowamidopropylamine Oxide, Tallowamine Oxide, Undecylenamidopropylamine Oxide und Wheat Germamidopropylamine Oxide. Bevorzugte Aminoxide sind beispielsweise Cocamidopropylamine Oxide (Cocoamidopropylaminoxid), aber auch N-Kokosalkyl-N,N-dimethylaminoxid, N-Talgalkyl-N,N-dihydroxyethylaminoxid, Myristylcetyldimethylaminoxid oder Lauryldimethylaminoxid.

Der Gehalt an Aminoxid beträgt in dem Handgeschirrspülmittel bevorzugt 1 bis 15 Gew.-% und vorzugsweise 2 bis 10 Gew.-%, jeweils bezogen auf das gesamte Handgeschirrspülmittel.

Zuckertenside sind bekannte oberflächenaktive Verbindungen, zu denen beispielsweise die Zuckertensidklassen der Alkylglucoseester, Aldobionamide, Gluconamide (Zuckersäureamide), Glycerinamide, Glyceringlykolipide, Polyhydroxyfettsäureamidzuckertenside (Zuckeramide) und Alkylpolyglykoside zählen. Im Rahmen der erfindungsgemäßen Lehre bevorzugte Zuckertenside sind die Alkylpolyglykoside und die Zuckeramide sowie deren Derivate, insbesondere ihre Ether und Ester. Bei den Ethern handelt es sich um die Produkte der Reaktion einer oder mehrerer, vorzugsweise einer, Zuckerhydroxygruppe mit einer eine oder mehrere Hydroxygruppen enthaltenden Verbindung, beispielsweise C₁₋₂₂-Alkoholen oder Glykolen wie Ethylen- und/oder Propylenglykol, wobei die Zuckerhydroxygruppe auch Polyethylenglykol- und/oder Polypropylenglykolreste tragen kann. Die Ester sind die Reaktionsprodukte einer oder mehrerer, vorzugsweise einer, Zuckerhydroxygruppe mit einer Carbonsäure, insbesondere einer C₆₋₂₂-Fettsäure.

Besonders bevorzugte Zuckeramide genügen der Formel R'C(O)N(R")[Z], in der R' für einen linearen oder verzweigten, gesättigten oder ungesättigten Acylrest, vorzugsweise einen linearen ungesättigten Acylrest, mit 5 bis 21, vorzugsweise 5 bis 17, insbesondere 7 bis 15, besonders bevorzugt 7 bis 13 Kohlenstoffatomen, R" für einen linearen oder verzweigten, gesättigten oder ungesättigten Alkylrest, vorzugsweise einen linearen ungesättigten Alkylrest, mit 6 bis 22, vorzugsweise 6 bis 18, insbesondere 8 bis 16, besonders bevorzugt 8 bis 14 Kohlenstoffatomen, einen C₁₋₅-Alkylrest, insbesondere einen Methyl-, Ethyl-, Propyl-, Isopropyl-, n-Butyl-, Isobutyl-, tert-Butyl- oder n-Pentylrest, oder Wasserstoff und Z für einen Zuckerrest, d.h. einen Monosaccharidrest, stehen. Besonders bevorzugte Zuckeramide sind die Amide der Glucose, die Glucamide, beispielsweise Lauroyl-methyl-glucamid.

Die Alkylpolyglykoside (APG) sind im Rahmen der erfindungsgemäßen Lehre besonders bevorzugte Zuckertenside und genügen vorzugsweise der allgemeinen Formel RⁱO(AO)ₐ[G]ₓ, in der Rⁱ für einen linearen oder verzweigten, gesättigten oder ungesättigten Alkylrest mit 6 bis 22, vorzugsweise 6 bis 18, insbesondere 8 bis 16, besonders bevorzugt 8 bis 14 Kohlenstoffatomen, [G] für einen glykosidisch verknüpften Zuckerrest und x für eine Zahl von 1 bis 10 sowie AO für eine Alkylenoxygruppe, z.B. eine Ethylenoxy- oder Propylenoxygruppe, und a für den mittleren Alkoxylierungsgrad von 0 bis 20 stehen. Hierbei kann die Gruppe (AO)ₐ auch verschiedene Alkylenoxyeinheiten enthalten, z.B. Ethylenoxy- oder Propylenoxyeinheiten, wobei es sich dann bei a um den mittleren Gesamtalkoxylierungsgrad, d.h. die Summe aus Ethoxylierungs- und Propoxylierungsgrad, handelt. Soweit nachfolgend nicht näher bzw. anders ausgeführt, handelt es sich bei den Alkylresten Rⁱ der APG um lineare ungesättigte Reste mit der angegebenen Zahl an Kohlenstoffatomen.

APG sind nichtionische Tenside und stellen bekannte Stoffe dar, die nach den einschlägigen Verfahren der präparativen organischen Chemie erhalten werden können. Die Indexzahl x gibt den Oligomerisierungsgrad (DP-Grad) an, d.h. die Verteilung von Mono- und Oligoglykosiden, und steht für eine Zahl zwischen 1 und 10. Während x in einer gegebenen Verbindung stets ganzzahlig sein muss und hier vor allem die Werte x = 1 bis 6 annehmen kann, ist der Wert x für ein bestimmtes Alkylglykosid eine analytisch ermittelte rechnerische Größe, die meistens eine gebrochene Zahl darstellt. Vorzugsweise werden Alkylglykoside mit einem mittleren Oligomerisierungsgrad x von 1,1 bis 3,0 eingesetzt. Aus anwendungstechnischer Sicht sind solche Alkylglykoside bevorzugt, deren Oligomerisierungsgrad kleiner als 1,7 ist und insbesondere zwischen 1,2 und 1,6 liegt. Als glykosidischer Zucker wird vorzugsweise Xylose, insbesondere aber Glucose verwendet.

Der Alkyl- bzw. Alkenylrest Rⁱ kann sich von primären Alkoholen mit 8 bis 18, vorzugsweise 8 bis 14 Kohlenstoffatomen ableiten. Typische Beispiele sind Capronalkohol, Caprylalkohol, Caprinalkohol und Undecylalkohol sowie deren technische Gemische, wie sie beispielsweise im Verlauf der Hydrierung von technischen Fettsäuremethylestern oder im Verlauf der Hydrierung von Aldehyden aus der ROELENschen Oxosynthese anfallen.

Vorzugsweise leitet sich der Alkyl- bzw. Alkenylrest Rⁱ aber von Laurylalkohol, Myristylalkohol, Cetylalkohol, Palmoleylalkohol, Stearylalkohol, Isostearylalkohol oder Oleylalkohol ab. Weiterhin sind Elaidylalkohol, Petroselinylalkohol, Arachidylalkohol, Gadoleylalkohol, Behenylalkohol, Erucylalkohol sowie deren technische Gemische zu nennen.

Besonders bevorzugte APG sind nicht alkoxyliert (a = 0) und genügen Formel RO[G]ₓ, in der R wie zuvor für einen linearen oder verzweigten, gesättigten oder ungesättigten Alkylrest mit 4 bis 22 Kohlenstoffatomen, [G] für einen glykosidisch verknüpften Zuckerrest, vorzugsweise Glucoserest, und x für eine Zahl von 1 bis 10, bevorzugt 1,1 bis 3, insbesondere 1,2 bis 1,6, stehen. Dementsprechend bevorzugte Alkylpolyglykoside sind beispielsweise C₈₋₁₀- und ein C₁₂₋₁₄-Alkylpolyglucosid mit einem DP-Grad von 1,4 oder 1,5, insbesondere C₈₋₁₀- Alkyl-1,5-glucosid und C₁₂₋₁₄-Alkyl-1,4-glucosid.

Das erfindungsgemäße Mittel kann zusätzlich ein oder mehrere kationische Tenside (Kationtenside; INCI Quaternary Ammonium Compounds) enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

Bevorzugte kationische Tenside sind die quaternären oberflächenaktiven Verbindungen, insbesondere mit einer Ammonium-, Sulfonium-, Phosphonium-, Jodonium- oder Arsoniumgruppe, die auch als antimikrobielle Wirkstoffe bekannt sind. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Besonders bevorzugte kationische Tenside sind die quaternären Ammoniumverbindungen (QAV; INCI Quaternary Ammonium Compounds) gemäß der allgemeinen Formel (R^{I})(R^{II})(R^{III})(R^{IV})N⁺ X⁻, in der R^{I} bis R^{IV} gleiche oder verschiedene C₁₋₂₂-Alkylreste, C₇₋₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere 12 bis 16, C-Atomen auf.

QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielsweise Benzalkoniumchlorid (N-Alkyl-N,N-dimethyl-benzylammoniumchlorid, CAS No. 8001-54-5), Benzalkon B (m,p-Dichlorbenzyl-dimethyl-C₁₂-alkylammoniumchlorid, CAS No. 58390-78-6), Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid, CAS No. 57-09-0), Benzetoniumchlorid (N,N-Dimethyl-N-[2-[2-[p-(1,1,3,3-tetramethylbutyl)phenoxy]ethoxy]ethyl]-benzylammoniumchlorid, CAS No. 121-54-0), Dialkyldimethylammoniumchloride wie Di-n-decyldimethyl-ammoniumchlorid (CAS No. 7173-51-5-5), Didecyldimethylammoniumbromid (CAS No. 2390-68-3), Dioctyl-dimethyl-ammoniumchlorid, 1-Cetylpyridiniumchlorid (CAS No. 123-03-5) und Thiazolinjodid (CAS No. 15764-48-1) sowie deren Mischungen. Bevorzugte QAV sind die Benzalkoniumchloride mit C₈-C₁₈-Alkylresten, insbesondere C₁₂-C₁₄-Alkyl-benzyl-dimethylammoniumchlorid. Eine besonders bevorzugte QAV ist das Kokospentaethoxymethylammoniummethosulfat (INCI PEG-5 Cocomonium Methosulfate; Rewoquat® CPEM).

Zur Vermeidung möglicher Inkompatibilitäten der kationischen Tenside mit den erfindungsgemäß enthaltenen anionischen Tensiden werden möglichst aniontensidverträgliches und/oder möglichst wenig kationisches Tensid eingesetzt oder in einer besonderen Ausführungsform der Erfindung gänzlich auf kationische Tenside verzichtet.

In einer bevorzugten Ausführungsform enthält das erfindungsgemäße Handgeschirrspülmittel zur Absenkung der Viskosität weiterhin ein oder mehrere wasserlösliche Salze. Es kann sich dabei um anorganische und/oder organische Salze handeln, in einer bevorzugten Ausführungsform enthält das Mittel dabei mindestens ein anorganisches Salz.

Erfindungsgemäß einsetzbare anorganische Salze sind dabei vorzugsweise ausgewählt aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle; weiterhin sind Ammoniumsalze einsetzbar. Besonders bevorzugt sind dabei Halogenide und Sulfate der Alkalimetalle; vorzugsweise ist das anorganische Salz daher ausgewählt aus der Gruppe umfassend Natriumchlorid, Kaliumchlorid, Natriumsulfat, Kaliumsulfat sowie Gemische derselben.

Bei den erfindungsgemäß einsetzbaren organischen Salzen handelt es sich insbesondere um farblose wasserlösliche Alkalimetall-, Erdalkalimetall-, Ammonium-, Aluminium- und/oder Übergangsmetallsalze der Carbonsäuren. Vorzugsweise sind die Salze ausgewählt aus der Gruppe umfassend Formiat, Acetat, Propionat, Citrat, Malat, Tartrat, Succinat, Malonat, Oxalat, Lactat sowie Gemische derselben.

Das erfindungsgemäße Handgeschirrspülmittel enthält in einer bevorzugten Ausführungsform 0,1 bis 10 Gew.-%, vorzugsweise 0,5 bis 7 Gew.-%, besonders bevorzugt 0,8 bis 5 Gew.-% mindestens eines wasserlöslichen Salzes. In einer besonders bevorzugten Ausführungsform werden dabei ausschließlich anorganische Salze eingesetzt.

Vorteilhafterweise sind in einer Ausführungsform der Erfindung in dem erfindungsgemäßen Mittel keine Buildersubstanzen vorhanden, welche Calcium-fällende Eigenschaften aufweisen. Dementsprechend wird ein Mittel bevorzugt, welches insbesondere keine Carbonat-haltigen Salze enthält.

Der Einsatz anderer Buildersubstanzen, vorzugsweise wasserlöslicher Buildersubstanzen kann hingegen von Vorteil sein.

Organische Buildersubstanzen, welche in Handgeschirrspülmittel vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), Methylglycindiessigsäure (MGDA) und deren Abkömmlinge sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g / mol.

Geeignete Polymere sind insbesondere Polyacrylate, die bevorzugt eine Molekülmasse von 1.000 bis 15.000 g / mol aufweisen. Aufgrund ihrer überlegenen Löslichkeit können aus dieser Gruppe wiederum die kurzkettigen Polyacrylate, die Molmassen von 1.000 bis 10.000 g / mol, und besonders bevorzugt von 1.000 bis 5.000 g / mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Bevorzugt werden allerdings lösliche Gerüststoffe, wie beispielsweise Citronensäure, oder Acrylpolymere mit einer Molmasse von 1.000 bis 5.000 g / mol in den flüssigen Handgeschirrspülmitteln eingesetzt.

Weiterhin können neben Wasser ein oder mehrere wasserlösliche organische Lösungsmittel enthalten sein. Das Lösungsmittel wird im Rahmen der erfindungsgemäßen Lehre nach Bedarf insbesondere als Hydrotropikum, Viskositätsregulator und/oder zusätzlicher Kältestabilisator eingesetzt. Es wirkt lösungsvermittelnd insbesondere für Tenside und Elektrolyt sowie Parfüm und Farbstoff und trägt so zu deren Einarbeitung bei, verhindert die Ausbildung flüssigkristalliner Phasen und hat Anteil an der Bildung klarer Produkte. Die Viskosität des erfindungsgemäßen Mittels verringert sich mit zunehmender Lösungsmittelmenge. Zuviel Lösungsmittel kann jedoch einen zu starken Viskositätsabfall bewirken.

Geeignete Lösungsmittel sind beispielsweise gesättigte oder ungesättigte, vorzugsweise gesättigte, verzweigte oder unverzweigte C₁₋₂₀-Kohlenwasserstoffe, bevorzugt C₂₋₁₅-Kohlenwasserstoffe, mit mindestens einer Hydroxygruppe und gegebenenfalls einer oder mehreren Etherfunktionen C-O-C, d.h. die Kohlenstoffatomkette unterbrechenden Sauerstoffatomen.

Bevorzugte Lösungsmittel sind die - gegebenenfalls einseitig mit einem C₁₋₆-Alkanol veretherten - C₂₋₆-Alkylenglykole und Poly-C₂₋₃-alkylenglykolether mit durchschnittlich 1 bis 9 gleichen oder verschiedenen, vorzugsweise gleichen, Alkylenglykolgruppen pro Molekül, beispielsweise PPG-2 Methyl Ether (Dipropylenglykolmonomethylether), wie auch die C₁₋₆-Alkohole, vorzugsweise Ethanol, n-Propanol oder iso-Propanol, insbesondere Ethanol.

Vorzugsweise ist das Lösungsmittel ausgewählt aus der Gruppe umfassend Methanol, Ethanol, Propanol, Isopropanol, Ethylenglykol, Propylenglykol, Glycerin sowie Gemischen derselben.

Ein besonders bevorzugtes und in Bezug auf die Stabilisierung der enzymatischen Handgeschirrspülmittel besonders wirksames organisches Lösungsmittel ist das Glycerin sowie das 1,2 Propylenglykol.

Im Hinblick auf die manuelle Anwendung der erfindungsgemäßen Mittel wird in einer bevorzugten Ausführungsform aber auf den Einsatz von organischen Lösungsmitteln verzichtet.

Als Lösungsvermittler insbesondere für Parfüm und Farbstoffe können außer den zuvor beschriebenen Lösungsmitteln beispielsweise auch Alkanolamine sowie Alkylbenzolsulfonate mit 1 bis 3 Kohlenstoffatomen im Alkylrest eingesetzt werden.

Neben den bisher genannten Komponenten können die erfindungsgemäßen Mittel weitere Inhaltsstoffe enthalten. Hierzu zählen beispielsweise weitere Tenside, Additive zur Verbesserung des Ablauf- und Trocknungsverhaltens, zur Einstellung der Viskosität, zur Stabilisierung sowie weitere in Handgeschirrspülmitteln übliche Hilfs- und Zusatzstoffe, etwa UV-Stabilisatoren, Parfüm, Perlglanzmittel (INCI Opacifying Agents; beispielsweise Glykoldistearat, z.B. Cutina® AGS der Fa. BASF, bzw. dieses enthaltende Mischungen, z.B. die Euperlane® der Fa. BASF), Farbstoffe, Korrosionsinhibitoren, Konservierungsmittel (z.B. das technische auch als Bronopol bezeichnete 2-Brom-2-nitropropan-1,3-diol (CAS 52-51-7), das beispielsweise als Myacide® BT oder als Boots Bronopol BT von der Firma Boots gewerblich erhältlich ist), organische Salze, Desinfektionsmittel, Bitterstoffe, antimikrobielle Wirkstoffe, pH-Stellmittel sowie Hautgefühl-verbessernde oder pflegende Additive (z.B. dermatologisch wirksame Substanzen wie Vitamin A, Vitamin B2, Vitamin B12, Vitamin C, Vitamin E, D-Panthenol, Sericerin, Collagen-Partial-Hydrolysat, verschiedene pflanzliche Protein-Partial-Hydrolysate, Proteinhydrolysat-Fettsäure-Kondensate, Liposome, Cholesterin, pflanzliche und tierische Öle wie z.B. Lecithin, Sojaöl, usw., Pflanzenextrakte wie z.B. Aloe Vera, Azulen, Hamamelisextrakte, Algenextrakte, usw., Allantoin, A.H.A.-Komplexe), die in Mengen von üblicherweise nicht mehr als 5 Gew.-% enthalten sein können.

Zur weiteren Verbesserung des Ablauf- und/oder Trocknungsverhaltens kann das erfindungsgemäße Mittel ein oder mehrere Additive aus der Gruppe der Tenside, der Polymere und der Buildersubstanzen (Builder) enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%, wobei wie oben beschrieben auf Calcium-fällende Buildersubstanzen weitestgehend verzichtet wird.

Als Additive geeignete Polymere sind insbesondere Maleinsäure-Acrylsäure-Copolymer-Na-Salz (Sokalan® CP 5), modifiziertes Polyacrylsäure-Na-Salz (Sokalan® CP 10), modifiziertes Polycarboxylat-Na-Salz (Sokalan® HP 25), Polyalkylenoxid, modifiziertes Heptamethyltrisiloxan (Silwet® L-77), Polyalkylenoxid, modifiziertes Heptamethyltrisiloxan (Silwet® L-7608) sowie Polyethersiloxane (Copolymere von Polymethylsiloxanen mit Ethylenoxid-/Propylenoxidsegmenten (Polyetherblöcken)), vorzugsweise wasserlösliche lineare Polyethersiloxane mit terminalen Polyetherblöcken wie Tegopren® 5840, Tegopren® 5843, Tegopren® 5847, Tegopren® 5851, Tegopren® 5863 oder Tegopren® 5878.

Als Additive geeignete Buildersubstanzen sind insbesondere Polyasparaginsäure-Na-Salz, Ethylendiamintriacetatkokosalkylacetamid (Rewopol® CHT 12), Methylglycindiessigsäure-Tri-Na-Salz (Trilon® ES 9964) und Acetophosphonsäure (Turpinal® SL). Mischungen mit tensidischen oder polymeren Additiven zeigen im Falle von Monawet® MO-84 R2W, Tegopren® 5843 und Tegopren® 5863 Synergismen. Der Einsatz der Tegopren-Typen 5843 und 5863 ist jedoch bei der Anwendung auf harte Oberflächen aus Glas, insbesondere Glasgeschirr, weniger bevorzugt, da diese Silikontenside auf Glas aufziehen können. In einer besonderen Ausführungsform der Erfindung wird auf die genannten Additive verzichtet.

Die für das erfindungsgemäße flüssige Mittel bevorzugte Viskosität liegt bei 20 °C und einer Scherrate von 30 min⁻¹ - gemessen mit einem Viskosimeter vom Typ Brookfield LV DV II und Spindel 31 - im Bereich von 10 bis 10.000 mPa·s, vorzugsweise 50 bis 5.000 mPa·s, insbesondere 100 bis 3.000 mPa·s, besonders bevorzugt 300 bis 2.000 mPa·s. Die Viskosität des erfindungsgemäßen Mittels kann - insbesondere bei einem geringen Tensidgehalt des Mittels - durch Verdickungsmittel erhöht und/oder - insbesondere bei einem hohen Tensidgehalt des Mittels - durch die enthaltenen wasserlöslichen anorganischen Salze sowie durch Lösungsmittel verringert werden.

Polymere Verdickungsmittel im Sinne der vorliegenden Erfindung sind die als Polyelektrolyte verdickend wirkenden Polycarboxylate, vorzugsweise Homo- und Copolymerisate der Acrylsäure, insbesondere Acrylsäure-Copolymere wie Acrylsäure-Methacrylsäure-Copolymere, und die Polysaccharide, insbesondere Heteropolysaccharide, sowie andere übliche verdickende Polymere.

Geeignete Polysaccharide bzw. Heteropolysaccharide sind die Polysaccharidgummen, beispielsweise Gummi arabicum, Agar, Alginate, Carrageene und ihre Salze, Guar, Guaran, Tragacant, Gellan, Ramsan, Dextran oder Xanthan und ihre Derivate, z.B. propoxyliertes Guar, sowie ihre Mischungen. Andere Polysaccharidverdicker, wie Stärken oder Cellulosederivate, können alternativ, vorzugsweise aber zusätzlich zu einem Polysaccharidgummi eingesetzt werden, beispielsweise Stärken verschiedensten Ursprungs und Stärkederivate, z.B. Hydroxyethylstärke, Stärkephosphatester oder Stärkeacetate, oder Carboxymethylcellulose bzw. ihr Natriumsalz, Methyl-, Ethyl-, Hydroxyethyl-, Hydroxypropyl-, Hydroxypropyl-methyl- oder Hydroxyethyl-methyl-cellulose oder Celluloseacetat.

Ein bevorzugtes polymeres Verdickungsmittel ist das mikrobielle anionische Heteropolysaccharid Xanthan Gum, das von Xanthomonas campestris und einigen anderen Species unter aeroben Bedingungen mit einem Molekulargewicht von 2-15×10⁶ produziert wird und beispielsweise von der Fa. Kelco unter dem Handelsnamen Keltrol® erhältlich ist, z.B. als cremefarbenes Pulver Keltrol® T (Transparent) oder als weißes Granulat Keltrol® RD (Readily Dispersable).

Als polymere Verdickungsmittel geeignete Acrylsäure-Polymere sind beispielsweise hochmolekulare mit einem Polyalkenylpolyether, insbesondere einem Allylether von Saccharose, Pentaerythrit oder Propylen, vernetzte Homopolymere der Acrylsäure (INCI Carbomer), die auch als Carboxyvinylpolymere bezeichnet werden. Solche Polyacrylsäuren sind u.a. von der Fa. BFGoodrich unter dem Handelsnamen Carbopol® erhältlich, z.B. Carbopol® 940 (Molekulargewicht ca. 4.000.000), Carbopol® 941 (Molekulargewicht ca. 1.250.000) oder Carbopol® 934 (Molekulargewicht ca. 3.000.000).

Besonders geeignete polymere Verdickungsmittel sind aber folgende Acrylsäure-Copolymere: (i) Copolymere von zwei oder mehr Monomeren aus der Gruppe der Acrylsäure, Methacrylsäure und ihrer einfachen, vorzugsweise mit C₁₋₄-Alkanolen gebildeten, Ester (INCI Acrylates Copolymer), zu denen etwa die Copolymere von Methacrylsäure, Butylacrylat und Methylmethacrylat (CAS 25035-69-2) oder von Butylacrylat und Methylmethacrylat (CAS 25852-37-3) gehören und die beispielsweise von der Fa. Rohm & Haas unter den Handelsnamen Aculyn® und Acusol® erhältlich sind, z.B. die anionischen nicht-assoziativen Polymere Aculyn® 33 (vernetzt), Acusol® 810 und Acusol® 830 (CAS 25852-37-3); (ii) vernetzte hochmolekulare Acrylsäurecopolymere, zu denen etwa die mit einem Allylether der Saccharose oder des Pentaerythrits vernetzten Copolymere von C₁₀₋₃₀-Alkylacrylaten mit einem oder mehreren Monomeren aus der Gruppe der Acrylsäure, Methacrylsäure und ihrer einfachen, vorzugsweise mit C₁₋₄-Alkanolen gebildeten, Ester (INCI Acrylates/C10-30 Alkyl Acrylate Crosspolymer) gehören und die beispielsweise von der Fa. BFGoodrich unter dem Handelsnamen Carbopol® erhältlich sind, z.B. das hydrophobierte Carbopol® ETD2623 und Carbopol® 1382 (INCI Acrylates/C10-30 Alkyl Acrylate Crosspolymer) sowie Carbopol® AQUA 30 (früher Carbopol® EX 473).

Der Gehalt an polymerem Verdickungsmittel beträgt üblicherweise nicht mehr als 8 Gew.-%, vorzugsweise zwischen 0,1 und 7 Gew.-%, besonders bevorzugt zwischen 0,5 und 6 Gew.-%, insbesondere zwischen 1 und 5 Gew.-% und äußerst bevorzugt zwischen 1,5 und 4 Gew.-%, beispielsweise zwischen 2 und 2,5 Gew.-%.

In einer bevorzugten Ausführungsform der Erfindung ist das Mittel jedoch frei von polymeren Verdickungsmitteln.

Zur Stabilisierung des erfindungsgemäßen Mittels, insbesondere bei hohem Tensidgehalt, können ein oder mehrere Dicarbonsäuren und/oder deren Salze zugesetzt werden, insbesondere eine Zusammensetzung aus Natriumsalzen der Adipin-, Bernstein- und Glutarsäure, wie sie z.B. unter dem Handelsnamen Sokalan® DSC erhältlich ist. Der Einsatz erfolgt hierbei vorteilhafterweise in Mengen von 0,1 bis 8 Gew.-%, vorzugsweise 0,5 bis 7 Gew.-%, insbesondere 1,3 bis 6 Gew.-% und besonders bevorzugt 2 bis 4 Gew.-%.

Eine Veränderung des Dicarbonsäure(salz)-Gehaltes kann - insbesondere in Mengen oberhalb 2 Gew.-% - zu einer klaren Lösung der Inhaltsstoffe beitragen. Ebenfalls ist innerhalb gewisser Grenzen eine Beeinflussung der Viskosität der Mischung durch dieses Mittel möglich. Weiterhin beeinflusst diese Komponente die Löslichkeit der Mischung. Diese Komponente wird besonders bevorzugt bei hohen Tensidgehalten eingesetzt, insbesondere bei Tensidgehalten oberhalb 30 Gew.-%.

Kann jedoch auf deren Einsatz verzichtet werden, so ist das erfindungsgemäße Mittel vorzugsweise frei von Dicarbonsäure(salze)n.

Als antibakterielle Komponente können erfindungsgemäße Mittel auch elementares Silber und/oder eine Silberverbindung enthalten.

Milchsäure hat auch den Vorteil, dass sie wie Benzoesäure oder auch Salicylsäure als pH-Regulator und/oder Puffer-Substanz die antibakterielle Wirkung des Silbers und/oder der Silberverbindung unterstützen bzw. verstärken kann

Der pH Wert des erfindungsgemäßen flüssigen Mittels kann aber auch mittels üblicher pH-Regulatoren, beispielsweise Säuren wie Mineralsäuren oder Citronensäure und/oder Alkalien wie Natrium- oder Kaliumhydroxid, eingestellt werden, wobei - insbesondere bei gewünschter Haut- und Handverträglichkeit - ein Bereich von 4 bis 10, vorzugsweise 5 bis 9 und insbesondere 5,5 bis 8,5, bevorzugt ist. Es hat sich gezeigt, dass das enzymhaltige Handgeschirrspülmittel, welches Kaliumsalze, insbesondere Kaliumacetat und/oder Kaliumchlorid, als Enzymstabilisator enthält, bei pH-Werten oberhalb von 7 zu besonders hohe Enzymaktivitäten und damit Enzymstabilitäten auch nach einer Lagerung der Mittel bei 30 °C führen. Erfindungsgemäße Mittel mit einem pH-Wert oberhalb von 7 werden daher ganz besonders bevorzugt. Zur Einstellung und/oder Stabilisierung des pH-Werts kann das erfindungsgemäße Mittel ein oder mehrere Puffer-Substanzen (INCI Buffering Agents) enthalten, üblicherweise in Mengen von 0,001 bis 5 Gew.-%, vorzugsweise 0,005 bis 3 Gew.-%, insbesondere 0,01 bis 2 Gew.-%, besonders bevorzugt 0,05 bis 1 Gew.-%, äußerst bevorzugt 0,1 bis 0,5 Gew.-%, beispielsweise 0,2 Gew.-%. Bevorzugt sind Puffer-Substanzen, die zugleich Komplexbildner oder sogar Chelatbildner (Chelatoren, INCI Chelating Agents) sind. Besonders bevorzugte Puffer-Substanzen sind die Citronensäure bzw. die Citrate, insbesondere die Natrium- und Kaliumcitrate, beispielsweise Trinatriumcitrat·2 H₂O und Trikaliumcitrat·H₂O.

Ein flüssiges Mittel kann weiterhin Hydrotrope enthalten. Hierbei handelt es sich um Löslichkeitsvermittler. Geeignete Hydrotrope sind beispielsweise Harnstoff, Butylglycol, oder aliphatische kurzkettige anionische oder amphotere Lösungsvermittler.

Eine besonders bevorzugte Ausführungsform der vorliegenden Erfindung betrifft ein Handgeschirrspülmittel enthaltend Kaliumacetat und eine Amylase-Zubereitung und/oder eine Protease-Zubereitung, wobei der Anteil der Kaliumionen an der Gesamtzusammensetzung von 0,02 bis 0,5 Gew.-% beträgt. In einer bevorzugten Weiterbildung enthält dieses manuelle Geschirrspülmittel außerdem 5 bis 40 Gew.-%, vorzugsweise 5 bis 30 Gew.& eines oder mehrerer anionischer Tenside und ein oder mehrere Betaine in einer Menge von 0,5 bis 15 Gew.-% (jeweils bezogen auf das Gesamtgewicht der Zusammensetzung). In einer weiteren Ausführungsform weist die Zusammensetzung einen pH Wert 5 bis 9, bevorzugt von 7 bis 8 auf.

In einer weiteren bevorzugten Ausführungsform enthält das manuelle Geschirrspülmittel mit Kaliumacetat und einer Amylase-Zubereitung und/oder einer Protease-Zubereitung und 0,02 bis 0,5 Gew.-% Kaliumionen, 8 bis 10 Gew.-% anionisches Tensid, 1 bis 2 Gew.-% amphoteres Tensid, 0,5 bis 1 Gew.-% Enzym, 1,0 bis 2,0 Gew.-% Salze, optional Farbstoff (z.B. 0,005 bis 0,015 Gew.-%), optional Parfüm (z.B. 0,1 bis 0,3 Gew.-%), optional Konservierungsmittel (z.B. 0,1 bis 0,3 Gew.-%), den Rest Wasser, und weist vorzugsweise einen pH-Wert von 5 bis 9, bevorzugt zwischen 7 und 8 auf.

In einer ganz besonders bevorzugten Ausführungsform enthält das manuelle Geschirrspülmittel mit Kaliumacetat und einer Amylase-Zubereitung und/oder einer Protease-Zubereitung und 0,2 Gew.-% Kaliumionen, 8,8 Gew.-% anionisches Tensid, 1,2 Gew.-% amphoteres Tensid, 0,8 Gew.-% Enzym, 2,0 Gew.-% Salze; optional Farbstoff (0,01 Gew.-%), optional Parfüm (0,2 Gew.-%), optional Konservierungsmittel (0,2 Gew.-%), den Rest Wasser, und weist vorzugsweise einen pH-Wert zwischen 5 und 9, bevorzugt zwischen 7 und 8 auf. In einer ganz speziellen Ausführungsform besteht das manuelle Geschirrspülmittel aus diesen Komponenten.

In einer weiteren bevorzugten Ausführungsform der vorliegenden Erfindung handelt es sich bei dem anionischen Tensid um ein Alkylethersulfat und bei dem amphoteren Tensid um ein oder mehrere Betaine.

Ein weiterer Gegenstand der vorliegenden Erfindung ist die Verwendung von Kaliumacetat zur Erhöhung der Stabilität von Enzymen, insbesondere von der Amylase, in flüssigen Wasch- oder Reinigungsmitteln, insbesondere in Handgeschirrspülmitteln nach einer der oben beschriebenen bevorzugten Ausführungsformen.

In einer Ausführungsform soll das erfindungsgemäße enzymhaltige und durch Kaliumacetat als Kaliumionenquelle stabilisierte Mittel zur Anwendung, also insbesondere zur Reinigung von verschmutzten Geschirr, in Form eines Schaums entweder direkt auf die zu reinigende Oberfläche oder auf einen Schwamm, ein Tuch, eine Bürste oder ein anderes, gegebenenfalls angefeuchtetes, Reinigungshilfsmittel aufgetragen werden. Zur Schaumerzeugung eignet sich in besonderer Weise ein manuell aktivierter Sprühspender, insbesondere ausgewählt aus der Gruppe umfassend Aerosolsprühspender, selbst Druck aufbauende Sprühspender, Pumpsprühspender und Triggersprühspender, insbesondere Pumpschaumspender, wie sie beispielsweise von der Firma Airspray, der Firma Taplast, der Firma Keltec oder auch der Daiwa Can Company angeboten werden. Neben Triggerflaschen eignen sich auch

Pumpsprühspender und Triggersprühspender mit einem Behälter aus Polyethylen, Polypropylen oder Polyethylenterephthalat. Solche Triggerflaschen werden beispielsweise von der Firma Afa-Polytec angeboten. Der Sprühkopf ist vorzugsweise mit einer Schaumdüse ausgestattet. Daneben kann das Mittel auch unter Zusatz eines geeigneten Treibmittels (z.B. n-Butan, ein Propan/Butan-Gemisch, Kohlendioxid, Stickstoff oder ein CO₂/N₂-Gemisch) in eine entsprechende Aerosolsprühflasche gefüllt werden. Ein solcher Sprühspender ist jedoch weniger bevorzugt.

Dementsprechend kann das erfindungsgemäße Mittel in Form eines Erzeugnisses aus dem erfindungsgemäßen Mittel und einem Sprüh- oder Schaumspender, insbesondere Pumpschaumspender, in Verkehr gebracht werden.

Zur manuellen Reinigung einer harten Oberfläche wird das erfindungsgemäße insbesondere flüssige Handgeschirrspülmittel entweder direkt, das heißt unverdünnt, beispielsweise mittels eines Schwamms, auf die zu reinigende Oberfläche aufgebracht und anschließend mit Wasser wieder entfernt.

Alternativ kann das erfindungsgemäße Handgeschirrspülmittel zunächst mit Wasser auf Konzentrationen von 1 : 1 bis 1 : 1000 verdünnt werden und anschließend wird die erhaltene Reinigungslösung mit der zu reinigenden Oberfläche in Kontakt gebracht.

Im Folgenden soll die vorliegende Erfindung durch Beispiele aufgezeigt werden. Diese Beispiele sind jedoch keinesfalls limitierend für den Gegenstand der vorliegenden Erfindung und dienen lediglich zur Veranschaulichung für den Fachmann.

### Beispiele

Das erfindungsgemäße manuelle Geschirrspülmittel E1 sowie das Vergleichsbeispiel V1 (siehe Tabelle 1; Mengenangaben in Gew.-%; Menge und Art an Parfüm, Farbstoff und Salzen waren in allen Rezepturen identisch) wurden 4 Wochen bei 30°C gelagert. Die Mittel waren direkt nach ihrer Herstellung wie auch nach der Lagerung klar. Der Farbeindruck des Farbstoffs hatte sich nicht verändert. Anschließend wurde die Restaktivität der eingesetzten Amylase mittels der Methode bestimmt, die in M. Lever, Carbohydrate Determination with 4-hydroxybenzoic acid hydrazide (PAHBAH): Effect of Bismuth on the Reaction, Anal. Biochem., 1977, 81, Seiten 21 bis 27 beschrieben ist. Die Messung des pH-Wertes erfolgte bei folgenden Messbedingungen: unverdünnt, gemessen bei 20 °C; die Einstellung wurde mittels Natriumhydroxid beziehungsweise Citronensäure vorgenommen.

**Tabelle 1: Vergleich erfindungsgemäßer Geschirrspülzusammensetzung E1 mit Vergleichsbeispiel V1. Es zeigte sich, dass E1 nach vierwöchiger Lagerung bei 30 °C eine Amylase-Restaktivität von 80 % aufwies, während bei dem Vergleichsbeispiel V1 keine Restaktivität mehr feststellbar war.**

| Inhaltsstoffe | E1 | V1 |
|---|---|---|
| C14-C16 Fettalkoholethersulfat mit 2 EO | 8,8 % | 8,8 % |
| Cocoamidopropylbetain | 1,2 % | 1,2 % |
| Parfüm | 0,2 % | 0,2 % |
| Farbstoff | 0,01 % | 0,01 % |
| Konservierungsmittel | 0,2 % | 0,2 % |
| Amylase Stainzyme 12L | 0,8 % | 0,8 % |
| Kaliumacetat | 0,2 % | 0,0 % |
| Salze | 2,0 % | 2,0 % |
| Wasser | auf 100 % | auf 100 % |
| pH-Wert | 8,0 | 8,0 |

Bei einer Lagerung des erfindungsgemäßen manuellen Geschirrspülmittels E1 bei 0 °C zeigten sich keine Trübungen oder Ausflockungen.

Damit konnte gezeigt werden, dass durch die erfindungsgemäße Verwendung von Kaliumionen in Handgeschirrspülmittelformulierungen eine ausgezeichnete Enzymstabilisierung bei gleichzeitiger Vermeidung von unerwünschten Trübungen bei Lagerung bei niedrigen Temperaturen erreicht werden kann.

## Patentansprüche

1. Flüssiges Wasch- oder Reinigungsmittel enthaltend Kaliumacetat und mindestens ein Enzym, **dadurch gekennzeichnet, dass** der Anteil der Kaliumionen an der Gesamtzusammensetzung von 0,02 bis 0,5 Gew.-% beträgt.

2. Wasch-und Reinigungsmittel nach Anspruch 1 enthaltend Amylase und/oder Protease.

3. Wasch- und Reinigungsmittel nach Anspruch 1 oder 2, **dadurch gekennzeichnet, dass** das Mittel ein Handgeschirrspülmittel ist.

4. Wasch- oder Reinigungsmittel gemäß mindestens einem der vorhergehenden Ansprüche, **dadurch gekennzeichnet, dass** der pH-Wert 5 bis 9 beträgt.

5. Wasch- oder Reinigungsmittel gemäß mindestens einem der vorhergehenden Ansprüche, **dadurch gekennzeichnet, dass** es ein oder mehrere Tenside enthält, vorzugsweise ein oder mehrere anionische Tenside und gegebenenfalls ein oder mehrere amphotere Tenside.

6. Wasch- oder Reinigungsmittel gemäß mindestens einem der vorhergehenden Ansprüche, **dadurch gekennzeichnet, dass** es weitere übliche Inhaltsstoffe von Wasch- oder Reinigungsmitteln enthält, vorzugsweise ausgewählt aus der Gruppe umfassend Salze, Builder, Lösungsmittel, weitere Tenside, Additive zur Verbesserung des Ablauf- und Trocknungsverhaltens, Verdickungsmittel, Additive zur Stabilisierung, Parfüm, Perlglanzmittel, Farbstoffe, UV-Stabilisatoren, Korrosionsinhibitoren, Konservierungsmittel, Desinfektionsmittel, Enzyme, pH-Stellmittel, Bitterstoffe, antimikrobielle Wirkstoffe, Hautgefühl-verbessernde oder pflegende Additive sowie Gemische derselben.

7. Verfahren zur Reinigung, insbesondere zur manuellen Reinigung harter Oberflächen, unter Einsatz eines Mittels gemäß mindestens einem der Ansprüche 1 bis 6.

8. Verwendung von Kaliumacetat zur Erhöhung der Stabilität von Enzymen, in Form einer Zusammensetzung enthaltend Kaliumacetat und mindestens ein Enzym, **dadurch gekennzeichnet, dass** der Anteil der Kaliumionen an der Gesamtzusammensetzung von 0,02 bis 0,5 Gew.-% beträgt, insbesondere von Amylase, in Form von flüssigen Reinigungsmitteln, insbesondere Handgeschirrspülmitteln.

## Claims

1. A liquid washing or cleaning agent containing potassium acetate and at least one enzyme, **characterized in that** the proportion of potassium ions in the total composition is from 0.02 to 0.5 wt.%.

2. The washing or cleaning agent according to claim 1, containing amylase and/or protease.

3. The washing or cleaning agent according to claim 1 or 2, **characterized in that** the agent is a hand dishwashing agent.

4. The washing or cleaning agent according to at least one of the preceding claims, **characterized in that** the pH is 5 to 9.

5. The washing or cleaning agent according to at least one of the preceding claims, **characterized in that** it contains one or more surfactants, preferably one or more anionic surfactants and optionally one or more amphoteric surfactants.

6. The washing or cleaning agent according to at least one of the preceding claims, **characterized in that** it contains further conventional ingredients of washing or cleaning agents, preferably selected from the group comprising salts, builders, solvents, additional surfactants, additives for improving the flow and drying behavior, thickening agents, additives for stabilization, perfume, pearlescing agents, dyes, UV stabilizers, corrosion inhibitors, preservatives, disinfectants, enzymes, pH adjusters, bitter substances, antimicrobial active ingredients, additives which improve skin feel or are nourishing, and mixtures thereof.

7. A method for cleaning, in particular for manually cleaning hard surfaces, using an agent according to at least one of claims 1 to 6.

8. The use of potassium acetate for increasing the stability of enzymes, in the form of a composition containing potassium acetate and at least one enzyme, **characterized in that** the proportion of potassium ions in the total composition is from 0.02 to 0.5 wt.%, in particular of amylase, in the form of liquid cleaning agents, in particular hand dishwashing agents.

## Revendications

1. Agent de lavage ou de nettoyage liquide contenant de l'acétate de potassium et au moins une enzyme, **caractérisé en ce que** la proportion d'ions potassium dans la composition totale va de 0,02 à 0,5 % en poids.

2. Agent de lavage ou de nettoyage selon la revendication 1 contenant une amylase et/ou une protéase.

3. Agent de lavage ou de nettoyage selon la revendication 1 ou la revendication 2, **caractérisé en ce que** l'agent est un détergent pour vaisselle à la main.

4. Agent de lavage ou de nettoyage selon au moins l'une des revendications précédentes, **caractérisé en ce que** la valeur du pH va de 5 à 9.

5. Agent de lavage ou de nettoyage selon l'une au moins des revendications précédentes, **caractérisé en ce qu'**il contient un ou plusieurs tensioactifs, de préférence un ou plusieurs tensioactifs anioniques et éventuellement un ou plusieurs tensioactifs amphotères.

6. Agent de lavage ou de nettoyage selon l'une au moins des revendications précédentes, **caractérisé en ce qu'**il contient d'autres ingrédients usuels des agents de lavage ou de nettoyage, choisis de préférence dans le groupe comprenant les sels, les adjuvants, les solvants, d'autres tensioactifs, les additifs pour l'amélioration du comportement à la vidange et au séchage, les agents épaississants, les additifs pour la stabilisation, un parfum, les agents nacrants, les colorants, les stabilisateurs d'UV, les inhibiteurs de corrosion, les agents de conservation, les agents désinfectants, les enzymes, les agents d'ajustement de pH, les substances amérisantes, les principes actifs antimicrobiens, les additifs soignant ou améliorant la sensation cutanée, ainsi que les mélanges de ceux-ci.

7. Procédé de nettoyage, en particulier de nettoyage manuel de surfaces dures, à l'aide d'un agent selon l'une au moins des revendications 1 à 6.

8. Utilisation d'acétate de potassium pour augmenter la stabilité d'enzymes, sous forme d'une composition contenant de l'acétate de potassium et au moins une enzyme, **caractérisée en ce que** la proportion d'ions potassium dans la composition totale va de 0,02 à 0,5 % en poids, en particulier d'amylase, sous forme d'agents de nettoyage liquides, en particulier de détergents pour vaisselle à la main.
